# EUROPEAN PATENT APPLICATION

(11) **EP 3 318 285 A1**
(43) Date of publication of application: **09.05.2018**
(21) Application number: 16197845.7
(22) Date of filing: 08.11.2016
(51) Int. Cl.: A61K 48/00, C07K 14/72

(54) **TREATMENT OF EYE DISEASE**

(71) Applicant: Oxford University Innovation Limited, Botley Oxford OX2 0JB (GB)
(72) Inventor: The designation of the inventor has not yet been filed
(74) Representative: D Young & Co LLP

(57) **Abstract**

An adeno-associated virus (AAV) vector for use in treating an eye disease, or improving or restoring vision, wherein the AAV vector is administered to a mammalian subject by subretinal injection, and wherein the AAV vector comprises a nucleotide sequence encoding melanopsin.

## Description

### FIELD OF THE INVENTION

The present invention relates to the gene therapy of eye diseases. More specifically, the present invention relates to methods using adeno-associated virus (AAV) vectors in the treatment of eye diseases (e.g. retinitis pigmentosa, RP), wherein the AAV vectors enable delivery of melanopsin to the eye.

### BACKGROUND TO THE INVENTION

In the developed world, most causes of blindness occur due to the loss of photoreceptors in the eye.

Retinitis pigmentosa (RP) is one such condition that is commonly caused by the progressive degeneration of rod photoreceptor cells. During progression of the disease, the cone photoreceptor cells and retinal pigment epithelium (RPE) may also degenerate.

RP is a phenotypically linked group of inherited retinal dystrophies that leads to gradual reduction in vision. Early symptoms of RP include deterioration of night and peripheral vision. As the disease progresses, detailed, central and colour vision may also be affected. The age of onset of RP symptoms is variable, but typically between 10 and 30, and the rate of deterioration varies between individuals. RP affects approximately 1 in 3000-4000 people.

In late stages of RP, most if not all photoreceptor cells may degenerate, giving rise to complete blindness. However, the eye itself and the optic nerve remain relatively intact, which provides the potential to restore eyesight if the light-sensing cells can be replaced.

Previous studies using an electronic retinal implant have successfully restored some vision to blind subjects (Stingl K. et al. (2013) Invest. Ophthalmol. Vis. Sci. 54: 7658-65). However, the restored vision with electronic approaches may be limited and there may be long term biocompatibility challenges when electronic devices are permanently implanted into the eye.

An alternative approach taken in the prior art was to convert neurons that are not naturally light sensitive into photoreceptors by the introduction of the light sensitive protein melanopsin (Lin B. et al. (2008) Proc. Natl. Acad. Sci. USA 105: 16009-14). However, the study in Lin et al. was limited to targeting retinal ganglion cells for the incorporation of exogenous melanopsin, based on the knowledge that melanopsin is endogenously expressed in a small subset of that type of cell. For example, Lin et al. considered the need to harness signalling systems within the cells themselves as an important factor in determining a successful outcome. Although Lin et al. reports the restoration of simple visual function in a mouse model, the results are limited to crude visual resolution. Furthermore, the effects reported in Lin et al. may simply be an augmentation of the natural responses in the existing light-sensitive ganglion cells that already express melanopsin.

There is currently no approved therapy to improve vision following the onset of RP. Accordingly, there remains a significant need for treatments of RP and other retinal dystrophies resulting in photoreceptor loss, in particular to restore visual function in severely affected patients in the late stages of the disease.

### SUMMARY OF THE INVENTION

The present inventors have surprisingly shown that targeting melanopsin to retinal bipolar and horizontal cells, which are next in line after photoreceptor degeneration, provides sensitivity to light.

The present inventors have discovered that it is possible, for example, to target the retinal bipolar and horizontal cells by injecting the AAV vector under the retina (i.e. using subretinal injection). Furthermore, the present inventors have unexpectedly shown that an AAV vector harbouring a Y733F mutant AAV8 capsid protein improves the transduction of the target cells in the retina.

Using the inventor's subretinal injection approach, the AAV vector is not put in contact with naturally melanopsin expressing cells and so all resulting light sensitivity must come from the AAV vector treatment.

Targeting the bipolar and horizontal cells is advantageous because these cells more closely represent the map that would previously have been formed by the photoreceptors. Although ganglion cells could be made light-sensitive, the axons of the ganglion cells run horizontally across the retina to the optic nerve head and it is difficult to predict how vision would be useful without some form of electronic interface to reprogram the nerve signals.

Furthermore, gene therapy using intravitreal injections may be more prone to inflammation as the vector more easily escapes from the eye. It would also be possible to apply higher concentrations of the vector using the subretinal approach.

Targeting the bipolar and horizontal cells has the additional advantage of feeding the light-sensitive signals at the most upstream part of the normal visual pathway.

Importantly, the present inventors have demonstrated that their approach provides for improvements in vision, in particular object recognition. The inventors' work extends significantly beyond studies of pupil light responses, which are subconscious responses that do not equate with vision. Pupil light responses are driven by ganglion cells and the midbrain, and are present in coma patients and people who are blind from retinitis pigmentosa. The pupil response can therefore be augmented without any perceptible improvement in vision. In contrast to this, the present inventors have carried out extensive further studies including cortical imaging experiments to show changes to cortical blood flow, and also behavioural and object recognition testing. These tests go far beyond pupil response studies, because they are driven by the visual cortex. They prove that the claimed methods can restore meaningful vision and object recognition, and not just augment an automated pupil reflex that may already be present.

In summary, the present inventors have discovered an unexpected method of gene therapy that may be applied for the treatment of end stage RP and other retinal dystrophies resulting in photoreceptor loss, for example in situations where no functioning rod and cone photoreceptor cells remain in the affected eye.

Accordingly, in one aspect the present invention provides an adeno-associated virus (AAV) vector for use in treating an eye disease, and/or improving or restoring vision, wherein the AAV vector is administered to a mammalian subject by subretinal injection, and wherein the AAV vector comprises a nucleotide sequence encoding melanopsin. Preferably, the nucleotide sequence encoding melanopsin is operably linked to an expression control sequence to promote expression of the melanopsin in cells of the eye of the subject.

In one embodiment, the AAV vector is for use in treating an eye disease. In another embodiment, the AAV vector is for use in improving or restoring vision, preferably in a subject suffering from an eye disease.

In another aspect, the present invention provides a method of treating an eye disease comprising administering an adeno-associated virus (AAV) vector to a mammalian subject by subretinal injection, wherein the AAV vector comprises a nucleotide sequence encoding melanopsin. Preferably, the nucleotide sequence encoding melanopsin is operably linked to an expression control sequence to promote expression of the melanopsin in cells of the eye of the subject.

In another aspect, the present invention provides a method of improving or restoring vision comprising administering an adeno-associated virus (AAV) vector to a mammalian subject by subretinal injection, wherein the AAV vector comprises a nucleotide sequence encoding melanopsin. Preferably, the nucleotide sequence encoding melanopsin is operably linked to an expression control sequence to promote expression of the melanopsin in cells of the eye of the subject.

In a preferred embodiment, object recognition is improved in the subject.

In another aspect, the present invention provides an adeno-associated virus (AAV) vector for use in improving or restoring object recognition, wherein the AAV vector is administered to a mammalian subject by subretinal injection, and wherein the AAV vector comprises a nucleotide sequence encoding melanopsin. Preferably, the nucleotide sequence encoding melanopsin is operably linked to an expression control sequence to promote expression of the melanopsin in cells of the eye of the subject. In a preferred embodiment, the subject is suffering from an eye disease.

In one embodiment, visual acuity is improved in the subject.

In another embodiment, the ability to recognise light and dark is improved in the subject. In another embodiment, the ability to navigate using visual cues is improved in the subject.

In one embodiment, the eye disease is a retinal dystrophy. In another embodiment, the eye disease is selected from retinitis pigmentosa, macular degeneration, age-related macular degeneration, diabetic retinopathy, cone-rod dystrophy or macular dystrophy. Preferably, the eye disease is retinitis pigmentosa.

In one embodiment, the eye to be treated has less than 50%, 40%, 30%, 20%, 10%, 5%, 4%, 3%, 2% or 1% of the functional rod and/or cone photoreceptor cells that were present prior to the onset of the disease.

In one embodiment, the eye to be treated substantially lacks rod and/or cone photoreceptor cells. The present invention advantageously may restore or improve vision in an eye in which substantially all rod and/or cone photoreceptor cells have degenerated due to disease. For example, the subject to be treated may be blind in the eye to be treated prior to administration of the AAV vector of the invention.

Preferably, the AAV vector is in the form of an AAV vector particle.

The AAV vector of the invention may comprise a genome and/or capsid of any serotype, provided that the vector is capable of mediating the expression of melanopsin in cells of the eye.

In one embodiment, the AAV vector comprises an AAV capsid protein of serotype 1, 2, 3, 4, 5, 6, 7, 8, 9, 10, or 11. In another embodiment, the AAV vector comprises an AAV capsid protein of serotype 2, 5 or 8. Preferably, the AAV vector comprises an AAV capsid protein of serotype 8. More preferably, the AAV vector is in the form of an AAV particle comprising an AAV8 Y733F mutant capsid.

In one embodiment, the AAV vector comprises an AAV serotype 1, 2, 3, 4, 5, 6, 7, 8, 9, 10, or 11 genome. In another embodiment, the AAV vector comprises an AAV serotype 2, 4, 5 or 8 genome. Preferably, the AAV vector comprises an AAV serotype 2 genome.

In one embodiment, the AAV vector comprises AAV2 ITRs.

In one embodiment, the AAV vector particle comprises an AAV2 genome and AAV2 capsid proteins (AAV2/2); an AAV2 genome and AAV5 capsid proteins (AAV2/5); or an AAV2 genome and AAV8 capsid proteins (AAV2/8). Preferably, the AAV vector particle comprises an AAV2 genome and AAV8 capsid proteins (AAV2/8), particularly preferably AAV8 Y733F mutant capsid proteins.

The AAV vector particle of the invention may be a chimeric, shuffled or capsid-modified derivative of one or more naturally occurring AAVs. In particular, the AAV vector particle may comprise capsid protein sequences from different serotypes, clades, clones or isolates of AAV within the same vector (i.e. pseudotyping). Thus, in one embodiment the AAV vector is in the form of a pseudotyped AAV vector particle.

Preferably, the melanopsin is expressed in bipolar and/or horizontal cells.

In one embodiment, the melanopsin is human melanopsin.

In another embodiment, the melanopsin-encoding nucleotide sequence is selected from the group consisting of:
(a) a nucleotide sequence having at least 70% identity to SEQ ID NO: 1 or 2; and
(b) a nucleotide sequence encoding an amino acid sequence having at least 70% identity to SEQ ID NO: 3 or 4,
wherein the protein encoded by the nucleotide sequence substantially retains the natural function of the protein represented by SEQ ID NO: 3 or 4.

In one embodiment, the nucleotide sequence encoding melanopsin is operably linked to a CBA promoter.

In one embodiment, the expression control sequence comprises a CBA promoter.

In one embodiment, the subretinal injection comprises the steps:
(a) administering a solution to the subject by subretinal injection in an amount effective to at least partially detach the retina to form a subretinal bleb, wherein the solution does not comprise the AAV vector; and
(b) administering a medicament composition by subretinal injection into the bleb formed by step (a), wherein the medicament comprises the AAV vector.

The AAV vector may, for example, be in a suspension at a concentration of about 1-2×10¹¹, 1-2×10¹² or 1-2×10¹³ genome particles (gp) per mL. Thus a dose of AAV vector of about 2×10¹⁰ gp may, for example, be administered by injecting about a 10 µL dose of AAV vector at a concentration of about 2×10¹² gp per mL. The skilled person is readily able to adjust the dose, volume and concentration of the AAV vector as necessary.

The volume of the AAV vector administered may be, for example, about 1-500 µL, for example about 10-500, 50-500, 100-500, 200-500, 300-500, 400-500, 50-250, 100-250, 200-250, 50-150, 1-100 or 1-10 µL. The volume may be, for example, about 1, 2, 5, 10, 50, 100, 150, 200, 250, 300, 350, 400, 450 or 500 µL. Preferably, the volume of the AAV vector composition injected is about 100 µL.

In one embodiment, the AAV vector is administered at a dosage of at least 2×10⁹, 2×10¹⁰, 2×10¹¹ or 2×10¹² gp per eye. In another embodiment, the AAV vector is administered at a dosage of about 1-2×10⁹, 1-2×10¹⁰, 1-2×10¹¹ or 1-2×10¹² gp per eye. Preferably, the AAV vector is administered at a dosage of about 2×10¹¹ gp per eye by subretinal injection.

### DESCRIPTION OF THE DRAWINGS

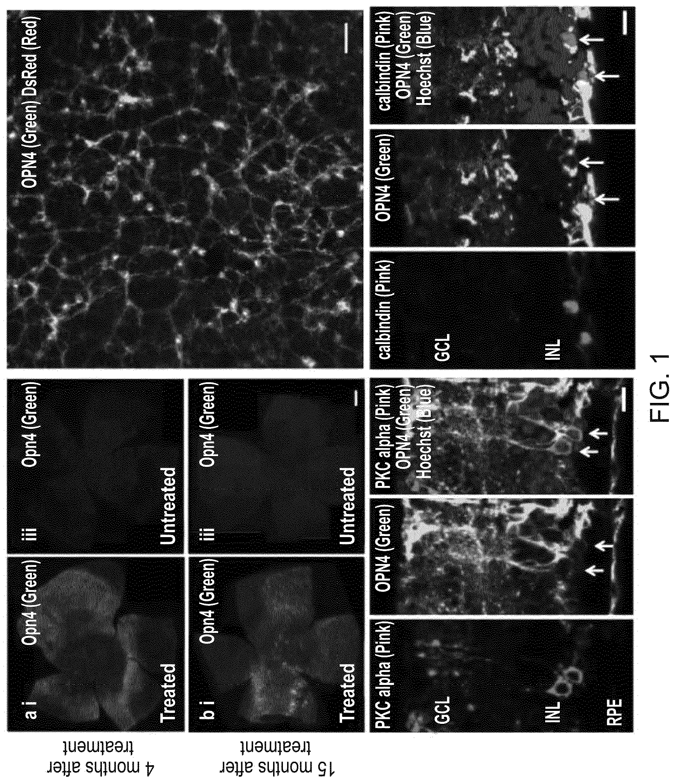

**Figure 1****: Long term expression of human melanopsin in the degenerate retina following subretinal delivery using an adeno-associated virus (AAV) vector**
   Widespread transduction of the retina of the *rd1* mouse (which has a rapid photoreceptor degeneration) immune-stained in green for human melanopsin - 4 months (a i) and 15 months (b i) after gene therapy. Images of control retinae are shown for comparison showing absence of human melanopsin at 4 months (a iii) and 15 months (b iii). The red label (DsRed) in the top right panel identifies transduced bipolar and horizontal cells on the subretinal surface. Appropriate membrane localisation of human melanopsin is also evident demonstrating the network of transduced cells. Immunostaining for PKC alpha identifying bipolar cells and calbindin identifying horizontal cells (bottom series of six panels) illustrates transduction of these cell types in the degenerate retina using the melanopsin vector.

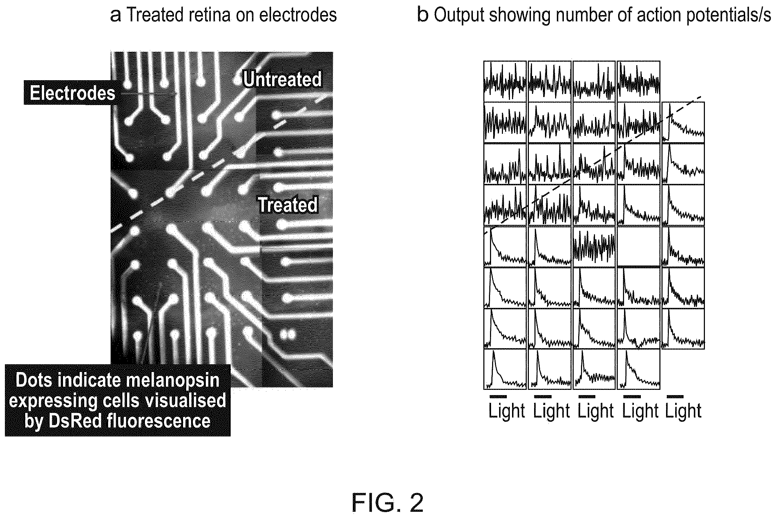

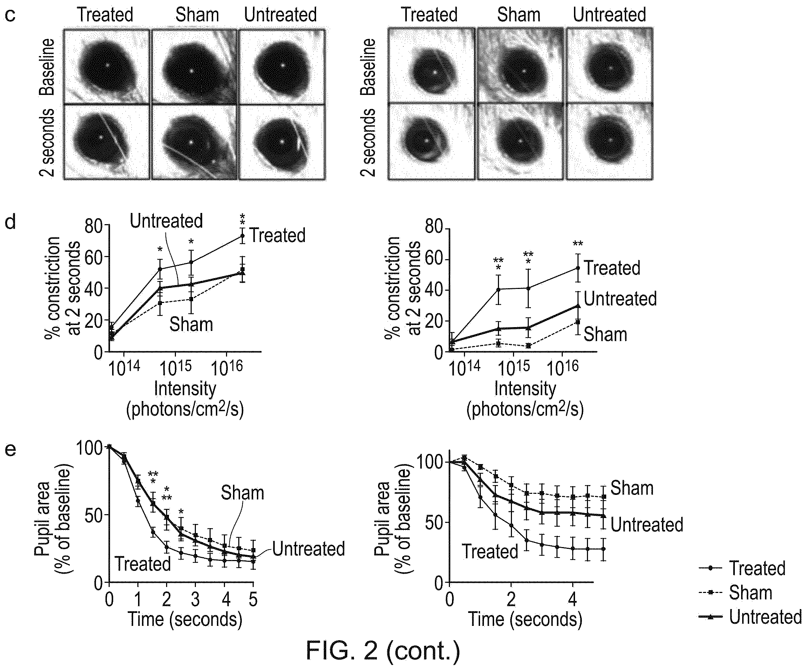

**Figure 2****: Human melanopsin expressed by an AAV vector in the degenerate retina is functional and able to drive light responses**
   Multi-electrode array (MEA) recordings from an ex vivo *rd1* mouse retina six months after treatment with melanopsin gene therapy. The lower half of the field below the yellow dotted line shows fluorescent dots (DsRed) which confirm the area of the retina transduced with the melanopsin expressing vector (a). Corresponding electrode recordings are shown in panel (b). The MEA recordings taken from retinal ganglion cells show action potentials corresponding to the regions of transduced retina below. These specific ganglion cell outputs could convey a retinotopic map of a visual image to the central visual centres.
   Pupil responses in blind mice two months (c, left panel) and twelve months (c, right panel) after transduction of the residual outer retina with 2×10⁹ genome particles of AAV vector with a Y733F mutant AAV8 capsid protein. The irradiance response curves (d) shows the mean and standard error of three cohorts of mice with significantly enhanced pupil constriction seen in the AAV vector treated group compared with sham-injected controls (*) and untreated retina (*). The response kinetics of the pupil constriction curve (e) following a stimulus of 2×10¹⁶ photons/cm²/s shows a slight delay (approximately 200 ms). The effects seen in the treated group at 2 months (d and e, left panels) are sustained at 12 months (d and e, right panels).

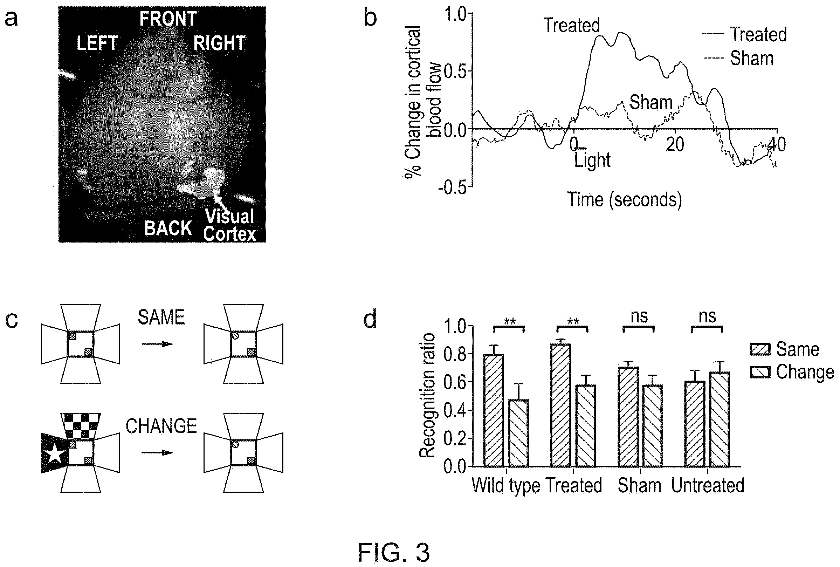

**Figure 3****: Human melanopsin expression in the degenerate retina may restore image-forming vision**
   The technique of laser speckle cortical imaging was used to assess blood flow changes in the visual cortex following a 2 second 480 nm light stimulus. A photograph of exposed skull is shown (a), indicating orientation and the location of the visual cortex. Activation in response to visual stimulation in a treated mouse is overlaid, with a large response corresponding to the right visual cortex, and a small response corresponding to the left visual cortex. Blood flow changes in the visual cortex were measured in treated (n=6) and sham-injected (n=5) mice. Mean responses in both groups are shown (b).
   Visual environment recognition test in mice treated with human melanopsin compared to controls at 12 months after injection. Schematic (c) illustrates the test where each animal has two trials where the visual environment is constant (same) or there is a change in visual environment during the task from a black and white patterned box to a plain white box (change). Measure of behaviour (d) with and without change in environment for each group demonstrating a significant difference in behaviour in wild type and treated mice when the visual environment is changed (** p<0.01, 2 way ANOVA with Tukey post hoc test).

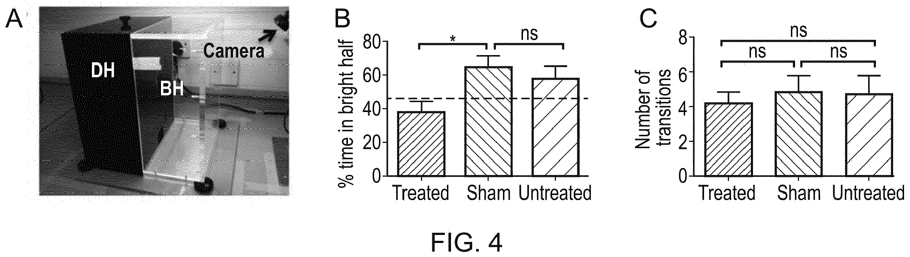

**Figure 4****: Visual function restored by human melanopsin expression in the degenerate retina was sustained at 13 months.**
   Behavioural light avoidance was assessed in response to a while light stimulus of 200 lux at ground level (A). There was a significant difference in time spent in the bright half (BH) of the chamber versus the dark half (DH) between mice treated with OPN4 vector thirteen months (37.65%±6.7) compared to sham-injected controls (64.66%±6.8) and untreated mice (57.65%±7.7) (B) (p=0.03 one way ANOVA; *p<0.05 with Tukey post hoc test; treated n=8, sham-injected n=9, untreated n=8). The number of transitions between compartments was similar in tested groups (p=0.88 one way ANOVA, ns=non significant with Tukey post hoc test) (C).

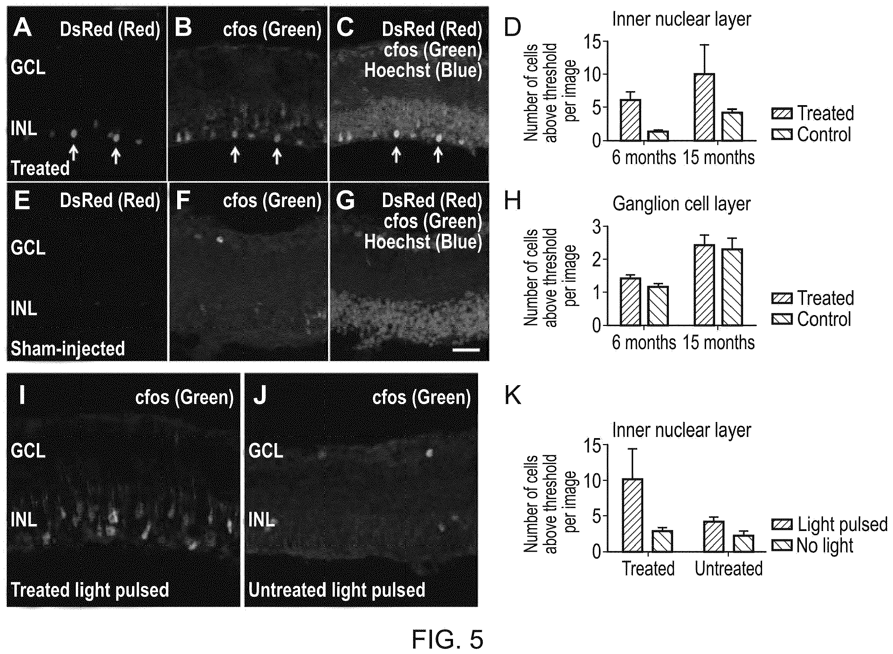

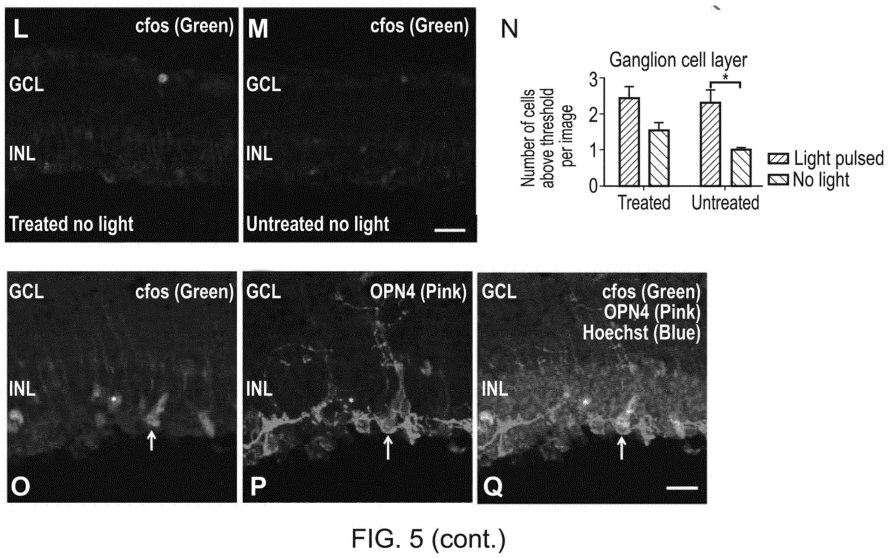

**Figure 5****: Light-induced cell depolarisation following subretinal OPN4 vector expression assessed by upregulation of c-Fos.**
   To assess whether ectopic expression of OPN4 was able to mediate cell depolarisation, c-Fos expression was assessed in treated rd1 mice versus age-matched controls at 6 months and 15 months after subretinal vector delivery. Mice were exposed to a 30 minute white light stimulus of 350 lux intensity and subsequently retinae collected and examined for c-Fos expression. Areas of vector expression were identified by DsRed fluorescence (red) (A). Antibody staining for c-Fos (green, B) and composite images showing overlay with Hoechst nuclear labelling (blue, C), demonstrate increased c-Fos expression in the inner nuclear layer (INL) of treated retina, (arrows indicate examples of cells expressing DsRed fluorescence and being labelled with c-Fos antibody). C-Fos expression was not widely seen in the INL of controls following a light-pulse (E-G, scale bar 25 µm). There was a significant effect of treatment (p=0.0197, 2 way ANOVA) but not of age (p=0.103) on the number of c-Fos positive cells in the INL following light pulsing (D; 6 month treated group n=303 c-Fos positive cells, N=5; control n=50 cells, N=4; 15 month treated group n=302 cells, N=3; control n=127, N=3). There was no effect of treatment (p=0.3419) but a significant effect of age (p=0.0004) on the number of c-Fos positive cells in the ganglion cell layer (GCL) following light pulsing (H; 6 month treated group n=72 c-Fos positive cells, N=5; control n=45 cells, N=4; 15 month treated group n=73 cells, N=3; control n=69, N=3).
   To confirm that c-Fos expression was upregulated by a light stimulus and not by other vector related effects, retinae from mice subjected to a light-pulse (I,J) were compared to those from mice handled in an identical manner but not exposed to light (L,M). There was a significant effect of light on the number of c-Fos positive cells in the GCL following a light stimulus (N; p=0.0038, 2 way ANOVA; untreated group p=0.02 Bonferroni post hoc test), but the overall effect of light did not reach significance examining cells in the INL (K; p=0.0739). * p<0.05, Bonferroni post hoc test.
   To evaluate whether c-Fos expressing cells in the INL expressed ectopic OPN4, retinae were co-labelled with c-Fos antibody (green) and OPN4 (purple) along with Hoechst nuclear stain (blue). This demonstrated c-Fos expression in OPN4 expressing cells (arrow, O-Q, scale bar 25 µm). A few adjacent c-Fos positive cells were identified that did not co-localise with OPN4 staining (*) suggestive of cell-to-cell signalling following a light stimulus.

### DETAILED DESCRIPTION OF THE INVENTION

In one aspect the present invention provides an adeno-associated virus (AAV) vector for use in treating an eye disease, and/or improving or restoring vision, wherein the AAV vector is administered to a mammalian subject by subretinal injection, and wherein the AAV vector comprises a nucleotide sequence encoding melanopsin.

### Retinitis pigmentosa (RP)

Retinitis pigmentosa (RP) is a phenotypically linked group of inherited retinal dystrophies which is commonly caused by the progressive degeneration of rod photoreceptor cells. The cone photoreceptor cells and retinal pigment epithelium (RPE) may also degenerate during progression of the disease. In late stage RP, most if not all rod and cone photoreceptors may be lost, which may result in blindness.

RP is characterised in clinical appearance by changes in the pigment of the retina, which may be accompanied by arteriolar attenuation and optic nerve atrophy. Changes in the retina may result from dispersion and aggregation of the retinal pigment. This may give rise to an appearance ranging from granular or mottled to distinctive focal aggregates resembling bone spicules. Black or dark brown star-shaped concentrations of pigment may appear. Furthermore, pigmentation limited to one quadrant of the retina, abnormalities which appear to be radiating out from the disc and changes associated with severe vasculopathy may be observed.

The method of treatment of RP described herein may, for example, be used to improve or restore visual function in subjects. Preferably, the treatment improves or restores visual function to an eye substantially lacking rod and cone photoreceptor cells.

Numbers of rods and cones can be estimated in the clinic by the skilled person using techniques such as adaptive optics, autofluorescence and optical coherence tomography (OCT) scans.

Visualisation of the appearance of a retina and assessment of visual function may also be readily carried out by the skilled person. Applicable visual function tests include, for example, best corrected visual acuity, visual field testing, microperimetry, colour vision, dark adaptometry, electroretinography and cone flicker fusion tests.

To confirm that a subject's optic nerve and central visual relays are intact, despite loss of photoreceptors, an electrically evoked phosphene (EEP) test may be carried out. This test confirms that potential subjects can experience light signals when the retina is stimulated with a current applied across a contact lens.

Preferably subjects to be treated will not exhibit significant loss of the retinal pigment epithelium (RPE), because melanopsin is dependent on the 11 cis-retinal, which is recycled from the all trans-retinal in the RPE.

### Structure of the eye

The method of the present invention requires the delivery of a medicament to a mammalian, preferably human eye.

The person skilled in the treatment of diseases of the eye will have a detailed and thorough understanding of the structure of the eye. However, the following structures of particular relevance to the present invention are described.

### Retina

The retina is the multi-layered membrane which lines the inner posterior chamber of the eye and senses an image of the visual world which is communicated to the brain via the optic nerve. In order from the inside to the outside of the eye, the retina comprises the layers of the neurosensory retina and retinal pigment epithelium, with the choroid lying outside the retinal pigment epithelium.

### Neurosensory retina and photoreceptor cells

The neurosensory retina harbours the photoreceptor cells that directly sense light. It comprises the following layers: internal limiting membrane (ILM); nerve fibre layer; ganglion cell layer; inner plexiform layer; inner nuclear layer; outer plexiform layer; outer nuclear layer (nuclei of the photoreceptors); external limiting membrane (ELM); and photoreceptors (inner and outer segments) of the rods and cones.

The skilled person will have a detailed understanding of photoreceptor cells. Briefly, photoreceptor cells are specialised neurons located in the retina that convert light into biological signals. Photoreceptor cells comprise rod and cone cells, which are distributed differently across the retina.

Rod cells are distributed mainly across the outer parts of the retina. They are highly sensitive and provide for vision at low light levels.

Cone cells are found across the retina, but are particular highly concentrated in the fovea, a pit in the neurosensory retina that is responsible for central high resolution vision. Cone cells are less sensitive than rod cells.

In addition to the rod and cone photoreceptor cells, the retina comprises a number of other neurons including bipolar cells, horizontal cells, amacrine cells and ganglion cells. The skilled person will have a detailed understanding of these cell types.

Briefly, bipolar cells are located in the retina adjacent to the photoreceptors. They transmit signals from the rod and cone cells to amacrine and/or ganglion cells. Bipolar cells may also synapse with horizontal cells.

Horizontal cells are located in the inner nuclear layer and are involved in signal processing and feedback to both photoreceptor and bipolar cells. They help integrate and regulate input from multiple photoreceptor cells.

Amacrine cells are located in the inner plexiform layer and act as a bridge between the photoreceptor pathway and ganglion cells.

Ganglion cells are located in the inner-most region of the retina. They transmit signals originating from the photoreceptor cells through the optic fibre layer to the origin of the optic nerve.

### Retinal pigment epithelium

The retinal pigment epithelium (RPE) is a pigmented layer of cells located immediately to the outside of the neurosensory retina. The RPE performs a number of functions, including transport of nutrients and other substances to the photoreceptor cells, and absorption of scattered light to improve vision.

### Choroid

The choroid is the vascular layer situated between the RPE and the outer sclera of the eye. The vasculature of the choroid enables provision of oxygen and nutrients to the retina.

### Melanopsin

Melanopsin is a membrane-bound light-sensitive protein similar to the rhodopsin present in photoreceptors. Melanopsin differs from rhodopsin in that it activates a second messenger system which is present in all neurons and not just rod and cone photoreceptors. Melanopsin is naturally located in a small subset of retinal ganglion cells. It contributes to pupil light response, and also functions to regulate circadian rhythm and the recognition of light-dark cycles.

Melanopsin may be encoded by the OPN4 gene. The melanopsin protein may also sometimes be referred to as OPN4.

In one embodiment of the present invention, the melanopsin is human melanopsin.

In one embodiment, the nucleotide sequence encoding melanopsin is the sequence deposited under NCBI Accession No. NM_033282.3.

In another embodiment, the nucleotide sequence encoding melanopsin is:

ATGAACCCTCCTTCGGGGCCAAGAGTCCCGCCCAGCCCAACCCAAGAGCCCAGCTGCATGGCCACCCCAG
CACCACCCAGCTGGTGGGACAGCTCCCAGAGCAGCATCTCCAGCCTGGGCCGGCTTCCATCCATCAGTCC
CACAGCACCTGGGACTTGGGCTGCTGCCTGGGTCCCCCTCCCCACGGTTGATGTTCCAGACCATGCCCAC
TATACCCTGGGCACAGTGATCTTGCTGGTGGGACTCACGGGGATGCTGGGCAACCTGACGGTCATCTATA
CCTTCTGCAGGAGCAGAAGCCTCCGGACACCTGCCAACATGTTCATTATCAACCTCGCGGTCAGCGACTT
CCTCATGTCCTTCACCCAGGCCCCTGTCTTCTTCACCAGTAGCCTCTATAAGCAGTGGCTCTTTGGGGAG
ACAGGCTGCGAGTTCTATGCCTTCTGTGGAGCTCTCTTTGGCATTTCCTCCATGATCACCCTGACGGCCA
TCGCCCTGGACCGCTACCTGGTAATCACACGCCCGCTGGCCACCTTTGGTGTGGCGTCCAAGAGGCGTGC
GGCATTTGTCCTGCTGGGCGTTTGGCTCTATGCCCTGGCCTGGAGTCTGCCACCCTTCTTCGGCTGGAGC
GCCTACGTGCCCGAGGGGTTGCTGACATCCTGCTCCTGGGACTACATGAGCTTCACGCCGGCCGTGCGTG
CCTACACCATGCTTCTCTGCTGCTTCGTGTTCTTCCTCCCTCTGCTTATCATCATCTACTGCTACATCTT
CATCTTCAGGGCCATCCGGGAGACAGGACGGGCTCTCCAGACCTTCGGGGCCTGCAAGGGCAATGGCGAG
TCCCTGTGGCAGCGGCAGCGGCTGCAGAGCGAGTGCAAGATGGCCAAGATCATGCTGCTGGTCATCCTCC
TCTTCGTGCTCTCCTGGGCTCCCTATTCCGCTGTGGCCCTGGTGGCCTTTGCTGGGTACGCACACGTCCT
GACACCCTACATGAGCTCGGTGCCAGCCGTCATCGCCAAGGCCTCTGCAATCCACAACCCCATCATTTAC
GCCATCACCCACCCCAAGTACAGGGTGGCCATTGCCCAGCACCTGCCCTGCCTGGGGGTGCTGCTGGGTG
TATCACGCCGGCACAGTCGCCCCTACCCCAGCTACCGCTCCACCCACCGCTCCACGCTGACCAGCCACAC
CTCCAACCTCAGCTGGATCTCCATACGGAGGCGCCAGGAGTCCCTGGGCTCGGAGAGTGAGGTGGGCTGG
ACACACATGGAGGCAGCAGCTGTGTGGGGAGCTGCCCAGCAAGCAAATGGGCGGTCCCTCTACGGTCAGG

GTCTGGAGGACTTGGAAGCCAAGGCACCCCCCAGACCCCAGGGACACGAAGCAGAGACTCCAGGGAAGAC
CAAGGGGCTGATCCCCAGCCAGGACCCCAGGATGTAG

(SEQ ID NO: 1)

In one embodiment, the nucleotide sequence encoding melanopsin is the sequence deposited under NCBI Accession No. NM_001030015.2.

In another embodiment, the nucleotide sequence encoding melanopsin is:

ATGAACCCTCCTTCGGGGCCAAGAGTCCCGCCCAGCCCAACCCAAGAGCCCAGCTGCATGGCCACCCCAG
CACCACCCAGCTGGTGGGACAGCTCCCAGAGCAGCATCTCCAGCCTGGGCCGGCTTCCATCCATCAGTCC
CACAGCACCTGGGACTTGGGCTGCTGCCTGGGTCCCCCTCCCCACGGTTGATGTTCCAGACCATGCCCAC
TATACCCTGGGCACAGTGATCTTGCTGGTGGGACTCACGGGGATGCTGGGCAACCTGACGGTCATCTATA
CCTTCTGCAGAGCTGTGCTTCGTGGAGTCACTGTGATGATGCAGAGCAGAAGCCTCCGGACACCTGCCAA
CATGTTCATTATCAACCTCGCGGTCAGCGACTTCCTCATGTCCTTCACCCAGGCCCCTGTCTTCTTCACC
AGTAGCCTCTATAAGCAGTGGCTCTTTGGGGAGACAGGCTGCGAGTTCTATGCCTTCTGTGGAGCTCTCT
TTGGCATTTCCTCCATGATCACCCTGACGGCCATCGCCCTGGACCGCTACCTGGTAATCACACGCCCGCT
GGCCACCTTTGGTGTGGCGTCCAAGAGGCGTGCGGCATTTGTCCTGCTGGGCGTTTGGCTCTATGCCCTG
GCCTGGAGTCTGCCACCCTTCTTCGGCTGGAGCGCCTACGTGCCCGAGGGGTTGCTGACATCCTGCTCCT
GGGACTACATGAGCTTCACGCCGGCCGTGCGTGCCTACACCATGCTTCTCTGCTGCTTCGTGTTCTTCCT
CCCTCTGCTTATCATCATCTACTGCTACATCTTCATCTTCAGGGCCATCCGGGAGACAGGACGGGCTCTC
CAGACCTTCGGGGCCTGCAAGGGCAATGGCGAGTCCCTGTGGCAGCGGCAGCGGCTGCAGAGCGAGTGCA
AGATGGCCAAGATCATGCTGCTGGTCATCCTCCTCTTCGTGCTCTCCTGGGCTCCCTATTCCGCTGTGGC
CCTGGTGGCCTTTGCTGGGTACGCACACGTCCTGACACCCTACATGAGCTCGGTGCCAGCCGTCATCGCC
AAGGCCTCTGCAATCCACAACCCCATCATTTACGCCATCACCCACCCCAAGTACAGGGTGGCCATTGCCC
AGCACCTGCCCTGCCTGGGGGTGCTGCTGGGTGTATCACGCCGGCACAGTCGCCCCTACCCCAGCTACCG
CTCCACCCACCGCTCCACGCTGACCAGCCACACCTCCAACCTCAGCTGGATCTCCATACGGAGGCGCCAG
GAGTCCCTGGGCTCGGAGAGTGAGGTGGGCTGGACACACATGGAGGCAGCAGCTGTGTGGGGAGCTGCCC
AGCAAGCAAATGGGCGGTCCCTCTACGGTCAGGGTCTGGAGGACTTGGAAGCCAAGGCACCCCCCAGACC
CCAGGGACACGAAGCAGAGACTCCAGGGAAGACCAAGGGGCTGATCCCCAGCCAGGACCCCAGGATGTAG

(SEQ ID NO: 2)

In one embodiment, the amino acid sequence of melanopsin is the sequence deposited under NCBI Accession No. NP_150598.1.

In another embodiment, the amino acid sequence of melanopsin is:

MNPPSGPRVPPSPTQEPSCMATPAPPSWWDSSQSSISSLGRLPSISPTAPGTWAAAWVPLPTVDVPDHAH
YTLGTVILLVGLTGMLGNLTVIYTFCRSRSLRTPANMFIINLAVSDFLMSFTQAPVFFTSSLYKQWLFGE
TGCEFYAFCGALFGISSMITLTAIALDRYLVITRPLATFGVASKRRAAFVLLGVWLYALAWSLPPFFGWS
AYVPEGLLTSCSWDYMSFTPAVRAYTMLLCCFVFFLPLLIIIYCYIFIFRAIRETGRALQTFGACKGNGE
SLWQRQRLQSECKMAKIMLLVILLFVLSWAPYSAVALVAFAGYAHVLTPYMSSVPAVIAKASAIHNPIIY
AITHPKYRVAIAQHLPCLGVLLGVSRRHSRPYPSYRSTHRSTLTSHTSNLSWISIRRRQESLGSESEVGW
THMEAAAVWGAAQQANGRSLYGQGLEDLEAKAPPRPQGHEAETPGKTKGLIPSQDPRM

(SEQ ID NO: 3)

In one embodiment, the amino acid sequence of melanopsin is the sequence deposited under NCBI Accession No. NP_001025186.1.

In another embodiment, the amino acid sequence of melanopsin is:

MNPPSGPRVPPSPTQEPSCMATPAPPSWWDSSQSSISSLGRLPSISPTAPGTWAAAWVPLPTVDVPDHAH
YTLGTVILLVGLTGMLGNLTVIYTFCRAVLRGVTVMMQSRSLRTPANMFIINLAVSDFLMSFTQAPVFFT
SSLYKQWLFGETGCEFYAFCGALFGISSMITLTAIALDRYLVITRPLATFGVASKRRAAFVLLGVWLYAL
AWSLPPFFGWSAYVPEGLLTSCSWDYMSFTPAVRAYTMLLCCFVFFLPLLIIIYCYIFIFRAIRETGRAL
QTFGACKGNGESLWQRQRLQSECKMAKIMLLVILLFVLSWAPYSAVALVAFAGYAHVLTPYMSSVPAVIA
KASAIHNPIIYAITHPKYRVAIAQHLPCLGVLLGVSRRHSRPYPSYRSTHRSTLTSHTSNLSWISIRRRQ
ESLGSESEVGWTHMEAAAVWGAAQQANGRSLYGQGLEDLEAKAPPRPQGHEAETPGKTKGLIPSQDPRM

(SEQ ID NO: 4)

The nucleotide sequence encoding melanopsin of the present invention may, for example, comprise a nucleotide sequence that has at least 70%, 80%, 90%, 95%, 96%, 97%, 98% 99% or 100% identity to SEQ ID NO: 1 or 2, wherein the protein encoded by the nucleotide sequence substantially retains the natural function of the protein represented by SEQ ID NO: 3 or 4.

The nucleotide sequence encoding melanopsin of the present invention may, for example, encode an amino acid sequence that has at least 70%, 80%, 90%, 95%, 96%, 97%, 98% 99% or 100% identity to SEQ ID NO: 3 or 4, wherein the amino acid sequence substantially retains the natural function of the protein represented by SEQ ID NO: 3 or 4.

Preferably, the nucleotide sequence of the present invention encodes a protein which assists in providing similar or higher light sensitivity or visual function when expressed in retinal bipolar and/or horizontal cells in a subject suffering from RP compared to the protein of SEQ ID NO: 3 or 4.

### Vectors

A vector is a tool that allows or facilitates the transfer of an entity from one environment to another.

The vectors used in the present invention may include a promoter for the expression of a polynucleotide and optionally a regulator of the promoter.

### Adeno-associated virus (AAV) vectors

The vector of the present invention is an adeno-associated virus (AAV) vector. Preferably, the AAV vector is in the form of an AAV vector particle.

Methods of preparing and modifying viral vectors and viral vector particles, such as those derived from AAV, are well known in the art and can be readily adapted by the skilled person to the required purpose.

The AAV vector may comprise an AAV genome or a derivative thereof.

An AAV genome is a polynucleotide sequence which encodes functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the vector of the invention is typically replication-deficient.

The AAV genome may be in single-stranded form, either positive or negative-sense, or alternatively in double-stranded form. The use of a double-stranded form allows bypass of the DNA replication step in the target cell and so can accelerate transgene expression.

The AAV genome may be from any naturally derived serotype, isolate or clade of AAV. Thus, the AAV genome may be the full genome of a naturally occurring AAV. As is known to the skilled person, AAVs occurring in nature may be classified according to various biological systems.

Commonly, AAVs are referred to in terms of their serotype. A serotype corresponds to a variant subspecies of AAV which, owing to its profile of expression of capsid surface antigens, has a distinctive reactivity which can be used to distinguish it from other variant subspecies. Typically, a virus having a particular AAV serotype does not efficiently cross-react with neutralising antibodies specific for any other AAV serotype.

AAV serotypes include AAV1, AAV2, AAV3, AAV4, AAV5, AAV6, AAV7, AAV8, AAV9, AAV10 and AAV11, and also recombinant serotypes, such as Rec2 and Rec3, recently identified from primate brain. Any of these AAV serotypes may be used in the present invention. Thus, in one embodiment of the present invention, the AAV vector particle is an AAV1, AAV2, AAV3, AAV4, AAV5, AAV6, AAV7, AAV8, AAV9, AAV10, AAV11, Rec2 or Rec3 AAV vector particle.

Preferred serotypes for use in the invention are AAV2, AAV4, AAV5 and AAV8.

Reviews of AAV serotypes may be found in Choi et al. (2005) Curr. Gene Ther. 5: 299-310 and Wu et al. (2006) Molecular Therapy 14: 316-27. The sequences of AAV genomes or of elements of AAV genomes including ITR sequences, rep or cap genes for use in the invention may be derived from the following accession numbers for AAV whole genome sequences: Adeno-associated virus 1 NC_002077, AF063497; Adeno-associated virus 2 NC_001401; Adeno-associated virus 3 NC_001729; Adeno-associated virus 3B NC_001863; Adeno-associated virus 4 NC_001829; Adeno-associated virus 5 Y18065, AF085716; Adeno-associated virus 6 NC_001862; Avian AAV ATCC VR-865 AY186198, AY629583, NC_004828; Avian AAV strain DA-1 NC_006263, AY629583; Bovine AAV NC_005889, AY388617.

AAV may also be referred to in terms of clades or clones. This refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

The skilled person can select an appropriate serotype, clade, clone or isolate of AAV for use in the present invention on the basis of their common general knowledge. For instance, the AAV5 capsid has been shown to transduce primate cone photoreceptors efficiently as evidenced by the successful correction of an inherited colour vision defect (Mancuso et al. (2009) Nature 461: 784-7).

The AAV serotype determines the tissue specificity of infection (or tropism) of an AAV virus. Accordingly, preferred AAV serotypes for use in AAVs administered to patients in accordance with the invention are those which have natural tropism for or a high efficiency of infection of target cells within the eye. In one embodiment, AAV serotypes for use in the present invention are those which infect bipolar and/or horizontal cells.

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. In preferred embodiments, one or more ITR sequences flank the nucleotide sequence encoding the melanopsin. The AAV genome typically also comprises packaging genes, such as rep and/or cap genes which encode packaging functions for an AAV particle. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle. Capsid variants are discussed below.

A promoter will be operably linked to each of the packaging genes. Specific examples of such promoters include the p5, p19 and p40 promoters (Laughlin et al. (1979) Proc. Natl. Acad. Sci. USA 76: 5567-5571). For example, the p5 and p19 promoters are generally used to express the rep gene, while the p40 promoter is generally used to express the cap gene.

As discussed above, the AAV genome used in the vector of the invention may therefore be the full genome of a naturally occurring AAV. For example, a vector comprising a full AAV genome may be used to prepare an AAV vector or vector particle in vitro. However, while such a vector may in principle be administered to patients, this will rarely be done in practice. Preferably the AAV genome will be derivatised for the purpose of administration to patients. Such derivatisation is standard in the art and the present invention encompasses the use of any known derivative of an AAV genome, and derivatives which could be generated by applying techniques known in the art. Derivatisation of the AAV genome and of the AAV capsid are reviewed in Coura and Nardi (2007) Virology Journal 4: 99, and in Choi et al. (2005) Curr. Gene Ther. 5: 299-310 and Wu et al. (2006) Molecular Therapy 14: 316-27.

Derivatives of an AAV genome include any truncated or modified forms of an AAV genome which allow for expression of a transgene from a vector of the invention in vivo. Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV genome. This allows for bypass of DNA replication in the target cell, and so enables accelerated transgene expression.

The one or more ITRs will preferably flank the nucleotide sequence encoding the melanopsin at either end. The inclusion of one or more ITRs is preferred to aid concatamer formation of the vector of the invention in the nucleus of a host cell, for example following the conversion of single-stranded vector DNA into double-stranded DNA by the action of host cell DNA polymerases. The formation of such episomal concatamers protects the vector construct during the life of the host cell, thereby allowing for prolonged expression of the transgene in vivo.

In preferred embodiments, ITR elements will be the only sequences retained from the native AAV genome in the derivative. Thus, a derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, in some embodiments, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the vector may be tolerated in a therapeutic setting.

Where a derivative comprises capsid proteins i.e. VP1, VP2 and/or VP3, the derivative may be a chimeric, shuffled or capsid-modified derivative of one or more naturally occurring AAVs. In particular, the invention encompasses the provision of capsid protein sequences from different serotypes, clades, clones, or isolates of AAV within the same vector i.e. pseudotyping.

Chimeric, shuffled or capsid-modified derivatives will be typically selected to provide one or more desired functionalities for the viral vector. Thus, these derivatives may display increased efficiency of gene delivery, decreased immunogenicity (humoral or cellular), an altered tropism range and/or improved targeting of a particular cell type compared to an AAV vector comprising a naturally occurring AAV genome, such as that of AAV2. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of a specific cell population, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N-and/or C-terminus of a capsid coding sequence.

The unrelated protein or peptide may advantageously be one which acts as a ligand for a particular cell type, thereby conferring improved binding to a target cell or improving the specificity of targeting of the vector to a particular cell population. An example might include the use of RGD peptide to block uptake in the retinal pigment epithelium and thereby enhance transduction of surrounding retinal tissues (Cronin et al. (2008) ARVO Abstract: D1048). The unrelated protein may also be one which assists purification of the viral particle as part of the production process, i.e. an epitope or affinity tag. The site of insertion will typically be selected so as not to interfere with other functions of the viral particle, e.g. internalisation or trafficking of the viral particle. The skilled person can identify suitable sites for insertion based on their common general knowledge. Particular sites are disclosed in Choi et al. (2005) Curr. Gene Ther. 5: 299-310.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

The vector of the invention may take the form of a nucleotide sequence comprising an AAV genome or derivative thereof and a sequence encoding the melanopsin transgene or a variant thereof.

The AAV particles of the invention include transcapsidated forms wherein an AAV genome or derivative having an ITR of one serotype is packaged in the capsid of a different serotype. The AAV particles of the invention also include mosaic forms wherein a mixture of unmodified capsid proteins from two or more different serotypes makes up the viral capsid. The AAV particle also includes chemically modified forms bearing ligands adsorbed to the capsid surface. For example, such ligands may include antibodies for targeting a particular cell surface receptor.

Thus, for example, the AAV particles of the invention include those with an AAV2 genome and AAV2 capsid proteins (AAV2/2), those with an AAV2 genome and AAV5 capsid proteins (AAV2/5) and those with an AAV2 genome and AAV8 capsid proteins (AAV2/8).

A preferred capsid protein for use in the present invention is the AAV8 Y733F mutant capsid (Petrs-Silva, H. et al. (2009) Mol. Ther. 17: 463-71).

Herein, amino acid residues within the AAV8 capsid are numbered following a convention whereby the N-terminal methionine of the example AAV8 capsid deposited under NCBI Accession No. YP_077180.1 (SEQ ID NO: 5) is assigned to be residue 1 (Y733 has been underlined in SEQ ID NO: 5, below).

MAADGYLPDWLEDNLSEGIREWWALKPGAPKPKANQQKQDDGRGLVLPGYKYLGPFNGLDKGEPVNAADA
AALEHDKAYDQQLQAGDNPYLRYNHADAEFQERLQEDTSFGGNLGRAVFQAKKRVLEPLGLVEEGAKTAP
GKKRPVEPSPQRSPDSSTGIGKKGQQPARKRLNFGQTGDSESVPDPQPLGEPPAAPSGVGPNTMAAGGGA
PMADNNEGADGVGSSSGNWHCDSTWLGDRVITTSTRTWALPTYNNHLYKQISNGTSGGATNDNTYFGYST
PWGYFDFNRFHCHFSPRDWQRLINNNWGFRPKRLSFKLFNIQVKEVTQNEGTKTIANNLTSTIQVFTDSE
YQLPYVLGSAHQGCLPPFPADVFMIPQYGYLTLNNGSQAVGRSSFYCLEYFPSQMLRTGNNFQFTYTFED
VPFHSSYAHSQSLDRLMNPLIDQYLYYLSRTQTTGGTANTQTLGFSQGGPNTMANQAKNWLPGPCYRQQR
VSTTTGQNNNSNFAWTAGTKYHLNGRNSLANPGIAMATHKDDEERFFPSNGILIFGKQNAARDNADYSDV
MLTSEEEIKTTNPVATEEYGIVADNLQQQNTAPQIGTVNSQGALPGMVWQNRDVYLQGPIWAKIPHTDGN
FHPSPLMGGFGLKHPPPQILIKNTPVPADPPTTFNQSKLNSFITQYSTGQVSVEIEWELQKENSKRWNPE
IQYTSNYYKSTSVDFAVNTEGVYSEPRPIGTRYLTRNL

(SEQ ID NO: 5)

Identities of individual residues and positions of mutations are described herein with reference to this numbering convention. A skilled person would readily be able to determine analogous positions in homologous proteins by performing a sequence alignment to this capsid protein.

### Promoters and regulatory sequences

The vector of the invention may also include elements allowing for the expression of the melanopsin transgene in vitro or in vivo. These may be referred to as expression control sequences. Thus, the vector typically comprises expression control sequences (e.g. comprising a promoter sequence) operably linked to the nucleotide sequence encoding the transgene.

By "operably linked", it is to be understood that the individual components are linked together in a manner which enables them to carry out their function substantially unhindered (e.g. a promoter may be operably linked to a nucleotide of interest to promote expression of the nucleotide of interest in a cell).

Any suitable promoter may be used, the selection of which may be readily made by the skilled person. The promoter sequence may be constitutively active (i.e. operational in any host cell background), or alternatively may be active only in a specific host cell environment, thus allowing for targeted expression of the transgene in a particular cell type (e.g. a tissue-specific promoter). The promoter may show inducible expression in response to presence of another factor, for example a factor present in a host cell. In any event, where the vector is administered for therapy, it is preferred that the promoter should be functional in the target cell background.

In some embodiments, it is preferred that the promoter shows retinal-cell specific expression in order to allow for the transgene to only be expressed in retinal cell populations. Thus, expression from the promoter may be retinal-cell specific, for example confined only to cells of the neurosensory retina and retinal pigment epithelium.

Preferred promoters include the chicken beta-actin (CBA) promoter, optionally in combination with a cytomegalovirus (CMV) enhancer element. An example promoter for use in the invention is a hybrid CAG promoter, for example the promoter used in previous clinical trials (MacLaren, R.E. et al. (2014) Lancet 383: 1129-37). A further example promoter for use in the invention has the sequence:

ATTGACGTCAATAATGACGTATGTTCCCATAGTAACGCCAATAGGGACTTTCCATTGACGTCAATGGGTG
GAGTATTTACGGTAAACTGCCCACTTGGCAGTACATCAAGTGTATCATATGCCAAGTACGCCCCCTATTG
ACGTCAATGACGGTAAATGGCCCGCCTGGCATTATGCCCAGTACATGACCTTATGGGACTTTCCTACTTG
GCAGTACATCTACGTATTAGTCATCGCTATTACCATGGTCGAGGTGAGCCCCACGTTCTGCTTCACTCTC
CCCATCTCCCCCCCCTCCCCACCCCCAATTTTGTATTTATTTATTTTTTAATTATTTTGTGCAGCGATGG
GGGCGGGGGGGGGGGGGGGCGCGCGCCAGGCGGGGCGGGGCGGGGCGAGGGGCGGGGCGGGGCGAGGCG

GAGAGGTGCGGCGGCAGCCAATCAGAGCGGCGCGCTCCGAAAGTTTCCTTTTATGGCGAGGCGGCGGCGG
CGGCGGCCCTATAAAAAGCGAAGCGCGCGGCGGGCGGGAGTCGCTGCGCGCTGCCTTCGCCCCGTGCCCC
GCTCCGCCGCCGCCTCGCGCCGCCCGCCCCGGCTCTGACTGACCGCGTTACTCCCACAGGTGAGCGGGCG
GGACGGCCCTTCTCCTCCGGGCTGTAATTAGCGCTTGGTTTAATGACGGCTTGTTTCTTTTCTGTGGCTG
CGTGAAAGCCTTGAGGGGCTCCGGGAGGGCCCTTTGTGCGGGGGGAGCGGCTCGGGGCTGTCCGCGGGG
GACGGCTGCCTTCGGGGGGGACGGGGCAGGGCGGGGTTCGGCTTCTGGCGTGTGACCGGCGGCTCTAGAG
CCTCTGCTAACCATGTTCATGCCTTCTTCTTTTTCCTACAGCTCCTGGGCAACGTGCTGGTTATTGTGCT
GTCTCATCATTTTGGCAAAGAATT

(SEQ ID NO: 6)

The vector of the invention may also comprise one or more additional regulatory sequences with may act pre- or post-transcriptionally. The regulatory sequence may be part of the native transgene locus or may be a heterologous regulatory sequence. The vector of the invention may comprise portions of the 5'-UTR or 3'-UTR from the native transgene transcript.

Regulatory sequences are any sequences which facilitate expression of the transgene, i.e. act to increase expression of a transcript, improve nuclear export of mRNA or enhance its stability. Such regulatory sequences include for example enhancer elements, post-transcriptional regulatory elements and polyadenylation sites. A preferred polyadenylation site is the Bovine Growth Hormone poly-A signal which may be as shown below:

TCGCTGATCAGCCTCGACTGTGCCTTCTAGTTGCCAGCCATCTGTTGTTTGCCCCTCCCCCGTGCCTTCC
TTGACCCTGGAAGGTGCCACTCCCACTGTCCTTTCCTAATAAAATGAGGAAATTGCATCGCATTGTCTGA
GTAGGTGTCATTCTATTCTGGGGGGTGGGGTGGGGCAGGACAGCAAGGGGGAGGATTGGGAAGACAATAG
CAGGCATGCTGGGGATGCGGTGGGCTCTATGGCTTCTGAGGCGGAAAGAACCAGCTGGGG

(SEQ ID NO: 7)

In the context of the vector of the invention, such regulatory sequences will be cis-acting. However, the invention also encompasses the use of trans-acting regulatory sequences located on additional genetic constructs.

A preferred post-transcriptional regulatory element for use in a vector of the invention is the woodchuck hepatitis post-transcriptional regulatory element (WPRE) or a variant thereof. An example sequence of the WPRE is shown below:

ATCAACCTCTGGATTACAAAATTTGTGAAAGATTGACTGGTATTCTTAACTATGTTGCTCCTTTTACGCT
ATGTGGATACGCTGCTTTAATGCCTTTGTATCATGCTATTGCTTCCCGTATGGCTTTCATTTTCTCCTCC
TTGTATAAATCCTGGTTGCTGTCTCTTTATGAGGAGTTGTGGCCCGTTGTCAGGCAACGTGGCGTGGTGT
GCACTGTGTTTGCTGACGCAACCCCCACTGGTTGGGGCATTGCCACCACCTGTCAGCTCCTTTCCGGGAC
TTTCGCTTTCCCCCTCCCTATTGCCACGGCGGAACTCATCGCCGCCTGCCTTGCCCGCTGCTGGACAGGG
GCTCGGCTGTTGGGCACTGACAATTCCGTGGTGTTGTCGGGGAAATCATCGTCCTTTCCTTGGCTGCTCG
CCTGTGTTGCCACCTGGATTCTGCGCGGGACGTCCTTCTGCTACGTCCCTTCGGCCCTCAATCCAGCGGA
CCTTCCTTCCCGCGGCCTGCTGCCGGCTCTGCGGCCTCTTCCGCGTCTTCGCCTTCGCCCTCAGACGAGT
CGGATCTCCCTTTGGGCCGCCTCCCCGC

(SEQ ID NO: 8)

The invention encompasses the use of any variant sequence of the WPRE which increases expression of the transgene compared to a vector without a WPRE. Preferably, variant sequences display at least 70% homology to SEQ ID NO: 8 over its entire sequence, more preferably 75%, 80%, 85%, 90% and more preferably at least 95%, 96% 97%, 98% or 99% homology to SEQ ID NO: 8 over its entire sequence.

Another regulatory sequence which may be used in a vector of the present invention is a scaffold-attachment region (SAR). Additional regulatory sequences may be readily selected by the skilled person.

### Method of administration

The AAV vector of the present invention is administered to the eye of a subject by subretinal injection.

The skilled person will be familiar with and well able to carry out individual subretinal injections.

### Subretinal injection

Subretinal injections are injections into the subretinal space, i.e. underneath the neurosensory retina. During a subretinal injection, the injected material is directed into, and creates a space between, the photoreceptor cell and retinal pigment epithelial (RPE) layers.

When the injection is carried out through a small retinotomy, a retinal detachment may be created. The detached, raised layer of the retina that is generated by the injected material is referred to as a "bleb".

The hole created by the subretinal injection must be sufficiently small that the injected solution does not significantly reflux back into the vitreous cavity after administration. Such reflux would be particularly problematic when a medicament is injected, because the effects of the medicament would be directed away from the target zone. Preferably, the injection creates a self-sealing entry point in the neurosensory retina, i.e. once the injection needle is removed, the hole created by the needle reseals such that very little or substantially no injected material is released through the hole.

To facilitate this process, specialist subretinal injection needles are commercially available (e.g. DORC 41G Teflon subretinal injection needle, Dutch Ophthalmic Research Center International BV, Zuidland, The Netherlands). These are needles designed to carry out subretinal injections.

Unless damage to the retina occurs during the injection, and as long as a sufficiently small needle is used, substantially all injected material remains localised between the detached neurosensory retina and the RPE at the site of the localised retinal detachment (i.e. does not reflux into the vitreous cavity). Indeed, the typical persistence of the bleb over a short time frame indicates that there is usually little escape of the injected material into the vitreous. The bleb may dissipate over a longer time frame as the injected material is absorbed.

Visualisations of the eye, in particular the retina, for example using optical coherence tomography, may be made pre-operatively.

### Two-step subretinal injection

The vector of the present invention may be delivered with increased accuracy and safety by using a two-step method in which a localised retinal detachment is created by the subretinal injection of a first solution. The first solution does not comprise the vector. A second subretinal injection is then used to deliver the medicament comprising the vector into the subretinal fluid of the bleb created by the first subretinal injection. Because the injection delivering the medicament is not being used to detach the retina, a specific volume of solution may be injected in this second step.

In one embodiment of the present invention, the AAV vector is delivered by:
(a) administering a solution to the subject by subretinal injection in an amount effective to at least partially detach the retina to form a subretinal bleb, wherein the solution does not comprise the AAV vector; and
(b) administering a medicament composition by subretinal injection into the bleb formed by step (a), wherein the medicament comprises the AAV vector.

The volume of solution injected in step (a) to at least partially detach the retina may be, for example, about 10-1000 µL, for example about 50-1000, 100-1000, 250-1000, 500-1000, 10-500, 50-500, 100-500, 250-500 µL. The volume may be, for example, about 10, 50, 100, 200, 300, 400, 500, 600, 700, 800, 900 or 1000 µL.

The volume of the medicament composition injected in step (b) may be, for example, about 10-500 µL, for example about 50-500, 100-500, 200-500, 300-500, 400-500, 50-250, 100-250, 200-250 or 50-150 µL. The volume may be, for example, about 10, 50, 100, 150, 200, 250, 300, 350, 400, 450 or 500 µL. Preferably, the volume of the medicament composition injected in step (b) is 100 µL. Larger volumes may increase the risk of stretching the retina, while smaller volumes may be difficult to see.

The solution that does not comprise the medicament (i.e. the "first solution" of step (a)) may be similarly formulated to the solution that does comprise the medicament, as described below. A preferred solution that does not comprise the medicament is balanced saline solution (BSS) or a similar buffer solution matched to the pH and osmolality of the subretinal space.

### Visualising the retina during surgery

Under certain circumstances, for example during end-stage retinal degenerations, identifying the retina is difficult because it is thin, transparent and difficult to see against the disrupted and heavily pigmented epithelium on which it sits. The use of a blue vital dye (e.g. Brilliant Peel^{®}, Geuder; MembraneBlue-Dual^{®}, Dorc) may facilitate the identification of the retinal hole made for the retinal detachment procedure (i.e. step (a) in the two-step subretinal injection method of the present invention) so that the medicament can be administered through the same hole without the risk of reflux back into the vitreous cavity.

The use of the blue vital dye also identifies any regions of the retina where there is a thickened internal limiting membrane or epiretinal membrane, as injection through either of these structures would hinder clean access into the subretinal space. Furthermore, contraction of either of these structures in the immediate post-operative period could lead to stretching of the retinal entry hole, which could lead to reflux of the medicament into the vitreous cavity.

### Pharmaceutical compositions and injected solutions

The medicaments, for example vectors, of the present invention may be formulated into pharmaceutical compositions. These compositions may comprise, in addition to the medicament, a pharmaceutically acceptable carrier, diluent, excipient, buffer, stabiliser or other materials well known in the art. Such materials should be non-toxic and should not interfere with the efficacy of the active ingredient. The precise nature of the carrier or other material may be determined by the skilled person according to the route of administration, i.e. subretinal injection.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001% may be used.

For injection at the site of affliction, the active ingredient may be in the form of an aqueous solution which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

### Method of treatment

It is to be appreciated that all references herein to treatment include curative, palliative and prophylactic treatment; although in the context of the present invention references to preventing are more commonly associated with prophylactic treatment. Treatment may also include arresting progression in the severity of a disease.

The treatment of mammals, particularly humans, is preferred. However, both human and veterinary treatments are within the scope of the present invention.

### Variants, derivatives, analogues, homologues and fragments

In addition to the specific proteins and nucleotides mentioned herein, the present invention also encompasses the use of variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question substantially retains its function. A variant sequence can be obtained by addition, deletion, substitution, modification, replacement and/or variation of at least one residue present in the naturally-occurring protein or polynucleotide.

The term "derivative" as used herein, in relation to proteins or polypeptides of the present invention includes any substitution of, variation of, modification of, replacement of, deletion of and/or addition of one (or more) amino acid residues from or to the sequence, providing that the resultant protein or polypeptide substantially retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions, provided that the modified sequence substantially retains the required activity or ability. Amino acid substitutions may include the use of non-naturally occurring analogues.

Proteins used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" as used herein means an entity having a certain homology with the wild type amino acid sequence and the wild type nucleotide sequence. The term "homology" can be equated with "identity".

A homologous sequence may include an amino acid sequence which may be at least 50%, 55%, 60%, 65%, 70%, 75%, 80%, 85% or 90% identical, preferably at least 95%, 96%, 97%, 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

A homologous sequence may include a nucleotide sequence which may be at least 50%, 55%, 60%, 65%, 70%, 75%, 80%, 85% or 90% identical, preferably at least 95%, 96%, 97%, 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye or, more usually, with the aid of readily available sequence comparison programs. These commercially available computer programs can calculate percent homology or identity between two or more sequences.

Percent homology may be calculated over contiguous sequences, i.e. one sequence is aligned with the other sequence and each residue in one sequence is directly compared with the corresponding residue in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical residues, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" of full length melanopsin are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the present invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Various preferred features and embodiments of the present invention will now be described by way of non-limiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. Each of these general texts is herein incorporated by reference.

### EXAMPLES

### Materials and methods

### Mice

C3H/HeNHsd-*Pde6b^{rd1}* (herein referred to as rd1) mice were purchased from Harlan Laboratories (Hillcrest, UK). Wild type mice of the same background strain that did not carry the rd mutation (non-rd C3H mice) were bred at the University of Oxford. Animals were housed under a 12 hour light (<100 lux) / dark cycle, with food and water available ad libitum. All procedures were performed under the approval of local and national ethical and legal authorities and in accordance with the Association for Research in Vision and Ophthalmology statements on the care and use of animals in ophthalmic research.

At the time of intraocular injection, mice were between 6 and 8 weeks old. For intraocular injection and laser speckle cortical imaging procedures, animals were anesthetized by intraperitoneal injection of 1 mg/kg medetomidine (Dormitor 1 mg/mL, Pfizer, Sandwich, UK) and 60 mg/kg ketamine (Ketaset 100 mg/mL, Fort Dodge, Southampton, UK). For procedures requiring pupil dilation, tropicamide 1% eye drops (Bausch & Lomb, Kingston-Upon-Thames, UK) and phenylephrine 2.5% eye drops (Bausch & Lomb, Kingston-Upon-Thames, UK) were used. All in vivo testing and tissue collection was performed during the day phase of the light/dark cycle, except for c-Fos induction.

### Intraocular injections

Intraocular injections were performed tangentially through the sclera with a 10 mm 34-gauge needle (Hamilton AG, Bonaduz, Switzerland) mounted on a 5 µL syringe (65 RN, Hamilton AG) under direct visual control using a surgical microscope (M620 Leica). A circular cover glass (∅6mm, VWR International, Lutterworth, UK) was applied onto the cornea with a carbomer coupling gel (Viscotears, Novartis, Frimley, UK) to ensure good visualization of the fundus. 1.5 µL viral vector solution (1.0 x 10¹² vg/mL, i.e. a dose of 1.5 x 10⁹ vg) was injected into each eye, and complete subretinal delivery was confirmed by direct visualization. Age-matched mice had a sham injection of phosphate buffered saline to account for any surgical effect of subretinal injection. After injection, the needle was left in position for an additional 20-30 seconds and then withdrawn quickly to minimize reflux and to allow self-sealing of the scleral tunnel.

### Viral vectors

Adeno-associated viral vectors (AAV) were produced using standard protocols. To produce the CMV.CBA-OPN4-IRES-DsRed construct: PCR SPLICE reactions (Davies, W.L. et al. (2007) BioTechniques 43: 785-789) were performed to join the CMV enhancer/CBA promoter (isolated from a pUf6.1 plasmid) to human OPN4. The IRES sequence from pIRES2-AcGFP plasmid (Clontech, USA) was joined to DsRed (from pSIREN-DNR-DsRed-Express plasmid, Clontech, USA) and the full construct created by a further SPLICE reaction in which Mfel and Notl restriction sites were inserted. PCR primers were designed manually and manufactured by Sigma-Aldrich (Dorset, UK). The full CMV.CBA-OPN4-IRES-DsRed construct was inserted into an AAV production plasmid using the Mfel and Notl restriction sites, containing wild type AAV2 inverted terminal repeat (ITRs), a Woodchuck hepatitis virus posttranscriptional regulatory element (WPRE) and bovine growth hormone polyadenylation signal, identical to the sequences used in the ongoing choroideremia gene therapy clinical trial (Maclaren, R.E. et al. (2014) Lancet 383: 1129-1137). After cloning, the presence of the construct was confirmed by full length sequencing in forward and reverse directions (Source Bioscience, Oxford, UK) and ITR integrity confirmed by Xmal digestion. The vector was custom packaged into the single capsid mutant rAAV2/8(Y733F) vector by Genedetect (Auckland, New Zealand). Viral titre was determined by qPCR to a region within OPN4, and was 1x10¹³ vg/mL.

### Retinal c-Fos expression

Mice were transferred into open top cages and placed in a light tight cabinet where they were maintained on a 12 hour light-dark cycle for 3 days. On the day of testing, four hours after normal lights off (i.e. ZT 16) mice were pulsed with a white light stimulus produced by LED light sources of 350 lux intensity measured at floor level for 30 minutes. Thirty minutes after the light stimulus ended, mice were sacrificed and eyes fixed in 4% paraformaldehyde, then processed as described below. Levels of c-Fos expression in the retina were assessed using immunohistochemistry. For analysis, images of retinal sections were taken at x20 magnification using identical acquisition settings on a confocal microscope (LSM710; Zeiss, Jena, Germany). Using Image J software, 8 bit images were analysed to perform cell counts and quantification of intensity of fluorescence (Protocol: subtract background 50 pixel rolling ball radius; threshold 0-20 and analyse particles).

### Multi electrode array recordings

Following enucleation, retina were dissected from eyes under dim red light (>610 nm) at room temperature in Ames media bubbled with 95% O₂ 5% CO₂ (pH 7.3) (Sigma Aldrich, UK). Retina were placed ganglion cell side down onto glass bottomed MEA chambers containing 60 electrodes each 30 µm in diameter and spaced 200 µm apart (Multi Channel Systems) and anchored in place with glass coated metal harps (ALA Scientific Instruments). MEA chambers were placed into the MEA recording device (MEA1060-Inv, Multi Channel Systems), fitted with a gas permeable perfusion manifold (ALA Scientific Instruments), and mounted onto the stage of an inverted Olympus IX71 microscope so that the recording electrodes were positioned in the microscope light path. Retina were continuously perfused with AMES media bubbled with 95% O₂ 5% CO₂ (pH 7.3) and maintained at 34°C using a combination of water bath heater (36°C), in-line perfusion heater (35°C), and base plate heater incorporated into the MEA system (34°C) to minimise temperature variations in the sample chamber. Recorded signals were collected, amplified and digitized at 25 kHz using MC Rack software (Multi Channel Systems).

Following isolation, retinae were perfused for 30 minutes in the dark prior to recording of light responses. For all recordings baseline activity was recorded for 60 seconds prior to light stimulation, followed by a 60 second light stimulus unless otherwise stated. Retinae were dark adapted for 20 minutes between recordings. A monochromatic light stimulus of 480 nm (bandwidth 20 nm) was generated by a Xenon arc light source with a slit monochromator (Cairn Optoscan) and delivered via a x10 microscope objective beneath the MEA chamber. Duration and wavelength of light stimuli were controlled via Metafluor software (Molecular Devices). Intensity of light stimuli were adjusted using neutral density filters (0 to 4 log units, Thor Labs) and controlled via an automated filter wheel placed into the microscope light path (Prior Scientific). Power of light stimuli (µW/cm2/s) were measured at the sample focal plane using an in-line power meter (PM160T, Thor Labs).

Data was analysed using MC Rack software (Multi Channel Systems). Action potentials were counted as electrical activity greater than 3 standard deviations of baseline activity and the number of action potentials in one second bins was plotted for analysis of responses. A light response was defined as a change in firing rate of 5 Hz or greater between the ten seconds prior to light onset compared to the ten seconds after light onset. For each recording, electrical activity was not seen in every electrode either due to the retinal explant not covering the electrode or a lack of good contact. Therefore the percentage of electrodes that showed electrical activity out of a potential 60 are stated per experiment. After measuring change in firing rate at 5 different light intensities (1.20 x10¹² photons/cm²/s, 1.33 x10¹³ photons/cm²/s, 1.37 x10¹⁴ photons/cm²/s, 3.99 x10¹⁴ photons/cm²/s and 1.34 x10¹⁵ photons/cm²/s) irradiance response curves for each electrode were fitted with sigmoidal dose response function (variable slope and EC50). The maximal response for these curves was set as response at the brightest light intensity and the sum of least squares was calculated to find the curve with the best fit to measured data.

### Laser speckle cortical imaging

Transcranial imaging was performed using the Speckle Contrast Imager (moorFLPI 2, Moor Instruments, UK). Mice were dark adapted for at least two hours prior to the procedure, which took place in a dark room. Following anaesthesia using intraperitoneal injection of 1 mg/kg medetomidine (Dormitor 1 mg/mL, Pfizer, Sandwich, UK) and 60 mg/kg ketamine (Ketaset 100 mg/mL, Fort Dodge, Southampton, UK) the temperature of the mouse was monitored and maintained via a rectal temperature probe and heatpad. Recordings were only performed when the core body temperature of the mouse was 36-37°C to ensure optimal conditions for recording physiological blood flow changes. Pupil dilation was administered as above, the head was fixed in a stereotaxic frame and a midline incision made in the scalp to reveal the cranium. Cranial sutures and landmarks could be clearly visualised following haemostasis, enabling the area anterior to lambda i.e. the location of the visual cortex to be identified. Two blue (480 nm) light emitting diodes (LEDs), each positioned on either side of the mouse's head were used as a light stimulus, of total intensity 2000 lux. A trial run was done without the mouse in situ to ensure that this light stimulus did not affect the image captured by the Speckle Contrast Imager.

Both eyes were stimulated simultaneously by a two second 480 nm light pulse with a one minute inter-stimulus interval. Ten stimuli were administered to each animal and the signal averaged to maximise signal to noise ratio. Treated mice (n=6) and sham-injected mice (n=5) were tested. Blood flow changes were captured using the Speckle Contrast Imager which uses a 785 nm laser with 50 mW laser power for measurement of blood flow. Images were acquired at 580 x 752 pixels at a rate of 5 frames per second. Data were processed using moorFLPI Review software and analysed with MATLAB R2012a version 7.14.0.739 (MathWorks, MA USA) and Origin Pro 8.6 (OriginLab, MA USA). The time series of cerebral blood flow changes were extracted from anatomically defined regions of interest corresponding to bilateral visual cortices. Percentage changes in cerebral blood flow were calculated for each trial with respect to pre-stimulus baseline values and trials were averaged for each mouse.

### Pupillometry

Mice that received a unilateral subretinal injection of OPN4 vector were compared with age matched mice that received a sham injection of phosphate buffered saline and with untreated controls. The consensual PLR was assessed to minimise any surgical effect on pupil constriction in treated eyes. The pupillometry apparatus used involved stimulating the left eye with light and recording the pupil response in the right (contralateral) eye using an infrared digital camera (Cohu, San Diego, USA). Experiments were done during the light phase of the light-dark cycle. Following dark adaptation for approximately two hours, an unanaesthetised mouse was held so that its right eye was in front of the infrared camera and left eye was in front of a Ganzfeld sphere connected to a 100 W xenon arc lamp (LOT-Oriel, Germany) via a light pipe. A 2 second white light stimulus was used and the Ganzfeld sphere ensured uniform illumination over the retina. Using neutral density filters, four different intensities of light were used with the dimmest stimulus being assessed first: 5.5 x 10¹³, 5.0 x 10¹⁴, 2.0 x 10¹⁵ and 2.0 x 10¹⁶ photons/cm²/s. An interval of at least twenty minutes was left between the different stimulus intensities for each mouse, with the animal kept in darkness between stimuli. For analysis, the pupil area was determined by manually identifying the pupil margin and subsequently measuring the area of the region of interest using Image J. Pupil area was measured at 2 seconds after the onset of light and normalised to baseline pupil size for generation of irradiance response curves. For generation of time course curves, pupil area was measured at 500 ms intervals and normalised to baseline.

### Behavioural light dark avoidance

Mice were tested in a 26 cm x 26 cm x 26 cm box, containing equally sized light and dark chambers connected by a 4 x 5 cm opening via which animals could move freely between chambers. The bright half of the box was illuminated by a white fluorescent light suspended above it, with intensity of 200 lux measured at floor level. Mice were light adapted and tested during the light phase of the light dark cycle. Animals were initially placed in the bright half facing away from the opening to the dark half, the lid of the chamber closed and movement recorded using a camera suspended facing the bright half (Logitech HD webcam, Logitech USA). A trial lasted three minutes and after this time, the animal was returned to its home cage and the testing apparatus dismantled and cleaned with 70% ethanol. Analysis was done using ANY-maze tracking software, and validated by comparing to manual video analysis. Time spent in the light chamber and number of four paw transitions between chambers (a measure of anxiety) were recorded. Mice that did not transition between chambers during the trial were excluded from analysis.

### Object recognition testing

This task was used to assess whether treated mice could detect a change in visual environment independent of ambient light levels. The basis of this test is an object recognition task comprising a sample phase, delay phase and test phase. In the sample phase, a mouse is placed into an open top box and allowed to explore two identical replicates of an object for a limited period of time. After a short delay, the mouse is placed into a further open top box with one replicate of the object previously encountered and another novel and distinct object. In this, the test phase, the animal is allowed to explore the familiar and novel objects. Animals often spend more time exploring the novel object, and this novelty preference is taken as evidence for recognition of the previously encountered object as being familiar (Ennaceur, A. et al. (1988) Behavioural Brain Research 31: 47-59; Bevins, R.A. et al. (2006) Nature Protocols 1: 1306-1311).

A visual context recognition task can be incorporated into this test to assess image-forming vision, based on the understanding that animals detect the background context in which they encounter an object. For example, when a familiar object is encountered in an unfamiliar background context with all other factors being constant, animals tend to perceive the object as novel and spend more time exploring it than if the object was encountered in its original context (Montgomery, K.C. (1953) Journal of Comparative and Physiological Psychology 46: 129-133; Dix, S.L. et al. (1999) Behavioural Brain Research 99: 191-200; Mumby, D.G. et al. (2002) Learning & Memory 9: 49-57; Whitt, E. et al. (2012) Journal of Experimental Psychology. Animal behavior processes 38: 74-83).

Animals were tested in the light phase of the light-dark cycle, in an environment illuminated by a white fluorescent light of 50 lux intensity at floor level. For the 'Same' environment condition, the (sample) phase of the test animals was carried out in a 30 cm x 30 cm white acrylic box containing two identical copies of an object placed in each corner. The mouse was allowed to freely explore these objects for the duration of the sample phase and movement was recorded using a camera suspended above the box (Sentient Mini-night vision CCTV camera, Maplin, UK). The mouse was then placed in its home cage for 5 minutes (delay phase). The mouse was subsequently placed in an identical copy of the 30 cm x 30 cm white acrylic box, with a new copy of the previously encountered object in one corner and a completely novel object in the other corner. For half the animals the novel object was placed in the top corner of the box, and for the others the novel object was placed at the bottom corner. The mouse was allowed to explore the objects for 3 minutes (test phase) and its movement recorded.

The mouse was then tested at least 4 days later in a similar test but with a change in visual environment during the test ('Change' condition). For this test, the sample phase was carried out in an open top box with a checkerboard pattern with alternating 4 cm × 4 cm black and white squares on one wall and a white symmetrical five-point star on a black background on another wall. The test phase was then carried out in an open top box made of white acrylic as previously, creating a change in visual environment between the sample and test phases. All other aspects of the object recognition test were identical under the 'Same' and 'Change' conditions, and the test arenas were illuminated by white light of 50 lux intensity throughout. For half of each group tested, the 'Same' condition was assessed first, and for the other half the 'Change' condition done first.

Videos of behaviour during the test were analysed using automated ANY-maze software and movement of the mouse's head was tracked. The outcome measure assessed was recognition ratio, expressed as n/(n+f), where f and n represent the average time per minute spent in contact with the familiar versus new objects (Mumby, D.G. et al. (2002) Learning & Memory 9: 49-57). A preference for the novel object would give a ratio of greater than 0.5, and preference for a familiar object would give a ratio less than 0.5. A ratio of 0.5 would suggest that the animal did not differentiate between familiar and novel objects during the test.

### Tissue collection and processing

For histological analysis, mice were sacrificed four months or fifteen months after subretinal injection. After enucleation, the cornea and lens were removed under direct visualisation with an operating microscope in 4% paraformaldehyde (PFA, Thermo Fisher, Loughborough, UK) in PBS. Following fixation overnight, the eyecups were cryoprotected using a 10-30% sucrose gradient. Eyecups were embedded in optimal cutting temperature (OCT) compound (Tissue-Tek, Sakura Finetek, The Netherlands), frozen on dry ice and stored at -80°C until sectioning. Eyecups were cryosectioned into 16 µm sections and affixed to poly-L-lysine coated glass slides (Polysine®; Thermo Scientific, Loughborough, UK). The sections were air-dried and then stored at -20°C until further histological processing.

### Immunocytochemistry and Immunohistochemistry

Retinal sections: After hydration and 3 x 5 min washes in 0.01 M PBS, retinal sections were blocked for 1 hour at room temperature in PBS + 0.1% Triton X-100 + 10% donkey serum. After 3 x 5 min washes, the sections were incubated at 4°C overnight with primary antibody, Table 1, and subsequently for 2 hours at room temperature with the species-appropriate secondary antibody, Table 2, both in PBS + 0.1% Triton X-100 + 1% serum. After each step, sections were rinsed for 2 x 5 min in PBS + 0.05% Tween20, followed by 1 x 5 min in PBS alone. All sections were counter-stained with Hoechst 33342 (Invitrogen) 1:5000 and mounted with an antifade reagent (Prolong Gold; Invitrogen).

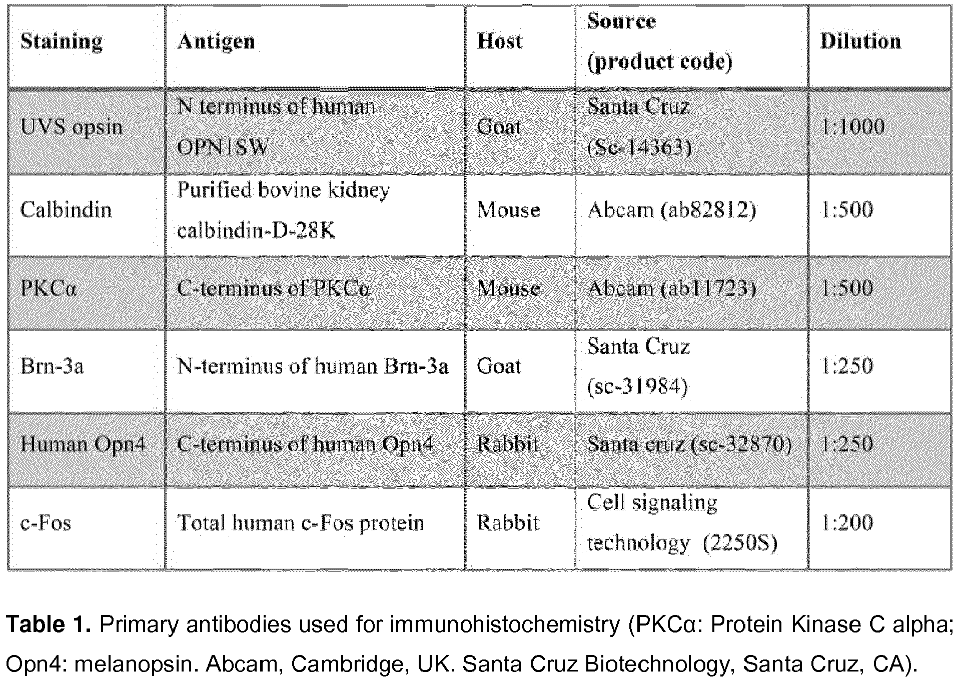

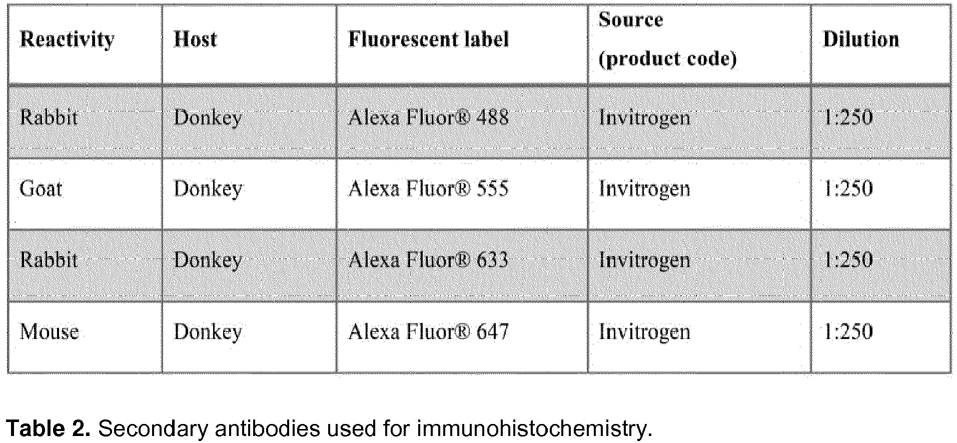

Retinal flatmounts: Following fixation overnight in 4% PFA, retinae were dissected from the eye and placed in 30% sucrose overnight. Following 3 x 10 minute washes in 0.01 M PBS + 1% Triton-X 100, retinae were blocked in PBS + 1% Triton X-100 + 10% donkey serum for two hours at room temperature. Subsequently retinae were incubated in primary antibody diluted in PBS + 1% Triton X-100 + 2.5% donkey serum at 4°C for three days. Flatmounts were rinsed three times for 30 minutes each in PBS + 0.2% Triton X-100 and incubated in secondary antibody diluted in PBS + 1% Triton X-100 + 2.5% donkey serum overnight at 4°C. Following three washes of 30 minutes duration in PBS + 0.2% Triton X-100, including Hoeschst 33342 nuclear stain (1:5000) during the final wash, retinae were mounted.

For immunohistochemistry performed using two primary antibodies: if the primary antibodies were raised in different species (e.g. rabbit and mouse) they were applied simultaneously as per the protocol above, with species-specific secondary antibodies also being applied at the same time. Staining for c-Fos and human melanopsin involved labelling with two primary antibodies raised in rabbit. In this case staining with the c-Fos antibody was carried out as above. Subsequently two further blocking steps were applied to saturate any unbound sites on the secondary antibody (rabbit anti-goat blocking antibody 1:200 for 2 hours at room temperature then Donkey anti-rabbit monovalent fragments 1:100 overnight at 4°C). Sections were then incubated with human melanopsin antibody and the appropriate secondary antibody as per the standard protocol.

### Confocal microscopy

Retinal sections were viewed on a confocal microscope (LSM710; Zeiss, Jena, Germany). Fluorescent cells were located using epifluorescence illumination before taking a series of overlapping XY optical sections, of approximately 0.5 µm thickness. The fluorescence of Hoechst, DsRed and Alexa-555, 568, 633 or 647 were sequentially excited using the appropriate wavelength laser. A stack was built to give an XY projection image and image processing was performed using Volocity (Perkin Elmer, Cambridge, UK) and Image J (Version 1.43, National Institute of Health, http://rsb.info.nih.gov/ij).

### Statistical analysis

Data for statistical analysis are presented as mean ± standard error of the mean (sem). The significance level was set as 0.05 in all cases. If two groups of data were normally distributed, a paired or unpaired t-test was used for comparison as appropriate. Otherwise a Mann-Witney test was performed. For more than two groups where the data was normally distributed, one or two way analysis of variance (ANOVA) was done depending on the number of factors to be analysed. Post hoc testing was done as specified in the data. For non-normally distributed data a Kruskal-Wallis test was carried out. All statistical analysis was done using Prism 6 for Mac OS X (San Diego, CA, USA).

### Example 1

The present inventors targeted the bipolar cells and horizontal cells of the retina, which are next in line after photoreceptor degeneration, for the introduction of exogenous melanopsin.

To achieve this, the inventors injected an AAV vector under the retina (i.e. by subretinal injection) in retinal degeneration (rd) mice, which have the human equivalent of end-stage RP (Fig. 1). The treated mice developed significantly increased pupil responses to light (Fig. 2) and the location of invoked electrical recordings correlated to the regions where bipolar and horizontal cells had been transduced (Fig. 2).

One of the advantages of targeting the bipolar and horizontal cells is that they more closely represent the map that would previously have been formed by the photoreceptors. Although ganglion cells could be made light-sensitive, the axons of the ganglion cells run horizontally across the retina to the optic nerve head and it is difficult to predict how vision would be useful without some form of electronic interface to reprogram the nerve signals.

Furthermore, gene therapy using intravitreal injections may be more prone to inflammation as the vector more easily escapes from the eye. It would also be possible to apply higher concentrations of the vector using the subretinal approach.

Targeting the bipolar and horizontal cells has the additional advantage of feeding the light-sensitive signals in at the most upstream part of the normal visual pathway.

In addition, the present inventors have improved the transduction of the retina using a novel type of AAV vector. The AAV vector has a mutation in one of the tyrosine residues in the capsid protein (AAV8 Y733F capsid mutant). This makes the vector considerably more efficient at expressing its gene product in cells of the retina.

This study used an AAV vector harbouring a melanopsin gene sequence. However, a gene for a red fluorescent protein (DsRed) was also incorporated into the vector, in order to study which cells were infected by the vector (Figs. 1 and 2). This also confirmed that the vector was still efficacious 15 months after the initial subretinal injection.

### Example 2

Histology confirmed the transduction of the outer retinal cells (bipolar and horizontal) that do not normally express melanopsin (Fig. 1).

Widespread transduction of the retina of the *rd1* mouse (which has a rapid photoreceptor degeneration) was observed by immune-staining for human melanopsin (Fig. 1 - 4 months (a i) and 15 months (b i) after gene therapy; images of control retinae are shown for comparison showing absence of human melanopsin at 4 months (a iii) and 15 months (b iii)). The red label (DsRed) in the top right panel of Fig. 1 identifies transduced bipolar and horizontal cells on the subretinal surface. Appropriate membrane localisation of human melanopsin was also observed, demonstrating the network of transduced cells.

Further immunostaining for PKC alpha (identifying bipolar cells) and calbindin (identifying horizontal cells) illustrated transduction of these cell types in the degenerate retina using the melanopsin vector (Fig. 1, bottom series of six panels).

### Example 3

### Methods

Capsid mutant AAV (rAAV2/8.Y733F) expressing human melanopsin was delivered via subretinal injection to the left eye of 15 Pde6b*rd1*/*rd1* mice. 9 mice were injected with phosphate-buffered saline (sham group) and 13 untreated mice were also included. 12 months after treatment, pupil responses were recorded from the right eye following stimulation of the left eye with a 2 second white light stimulus of varying intensity.

### Results

12 months after subretinal injection, a large area of retina in each treated eye expressed human melanopsin, as confirmed by immunohistochemistry. At a light intensity of 2×10¹⁵ photons/cm²/s, which was in the mid-range of the light intensities tested, there was significantly more pupil constriction in the treated group (mean constriction 36.0% ± 7.06) versus the sham treated group (3.1% ± 1.45) and the untreated group (15.6% ± 6.61); treated versus sham p=0.0026, treated versus untreated p=0.038 2 way ANOVA, Tukey's post hoc test.

### Conclusions

Human melanopsin can be successfully delivered via subretinal injection to the mouse degenerate retina using capsid mutant AAV, with a sustained high level of gene expression seen after 12 months. The pupil light response in treated animals was significantly improved compared to sham-injected and untreated mice, providing evidence that human melanopsin mediated photosensitivity can restore visual function in the degenerate retina.

### Example 4

The present inventors also studied visually evoked readings from the cortex of blind *rd1* mice after transduction with an AAV vector harbouring the melanopsin gene. The readings showed changes in blood flow, a biomarker of increased neuronal activity, in a part of the brain that is not a target of the melanopsin expressing ganglion cells normally present in the eye. This study provides additional proof of activation of a novel pathway.

Further experiments carried out by the present inventors related to object recognition (another functional test) and showed a statistically significant improvement following administration of the AAV vector. Object recognition would most likely require cortical activation.

The technique of laser speckle cortical imaging was used to assess blood flow changes in the visual cortex following a 2 second 480 nm light stimulus. A photograph of exposed skull is shown in Fig. 3(a) which indicates orientation and the location of the visual cortex. Activation in response to visual stimulation in a treated mouse is overlaid, with a large response corresponding to the right visual cortex, and a small response corresponding to the left visual cortex. Blood flow changes in the visual cortex were measured in treated (n=6) and sham-injected (n=5) mice. Mean responses in both groups are shown in Fig. 3(b).

Visual environment recognition tests were also carried out in mice treated with human melanopsin compared to controls at 12 months after injection. Schematic Fig. 3(c) illustrates tests where each animal has two trials where the visual environment is constant (same); or where there is a change in visual environment during the task from a black and white patterned box to a plain white box (change). Measure of behaviour (Fig. 3(d)) with and without change in environment for each group demonstrated a significant difference in behaviour in wild type and treated mice when the visual environment is changed (** p<0.01, 2 way ANOVA with Tukey post hoc test).

### Example 5

To assess whether information generated by ectopic OPN4 expression could drive visually guided behaviour, animals were assessed using a behavioural light avoidance assay (Semo, M. et al. (2010) PLoS One 5, e15009; Singh, M.S. et al. (2013) Proc. Natl. Acad. Sci. USA 110: 1101-1106) based on the natural preference of mice to avoid brightly lit environments (Fig. 4A). Thirteen months after OPN4 vector delivery, there was a significant difference in time spent in the brightly lit chamber between groups (p=0.03, Fig. 4B), with the vector treated group showing behaviour closest to wild type mice. This was not due to a difference in general or anxiety-related locomotor activity since the number of transitions between bright and dark chambers was similar between groups (p=0.88, Fig. 4C). Functional effects were unlikely to be mediated by residual cones in the rd1 mouse, since these cells were morphologically abnormal and similar numbers of cells remained between groups (p=0.123). Furthermore, all mice were homozygous for the gpr179 mutation, excluding any input from residual photoreceptors in mediating ON bipolar cell depolarisation via the mGlur6 cascade (Ray, T.A. et al. (2014) J. Neurosci. 34: 6334-6343).

### Example 6

Expression of the 'immediate early' gene c-Fos, is a marker of cellular depolarisation and has been used to monitor melanopsin driven light responses in ipRGCs (Sheng, M. et al. (1990) Neuron 4: 571-582; Semo, M. et al. (2003) The European Journal of Neuroscience 18: 3007-3017). Comparison of light induced c-Fos expression in the inner nuclear layer (INL) of vector treated versus untreated retina showed a 2.5 fold increase in the number of c-Fos positive cells in treated eyes (p=0.0197, Figure 5A-G). High levels of co-localisation were observed for DsRed and c-Fos (Figure 5A-C), and human OPN4 and c-Fos (Figure 5O-Q) confirming light-induced depolarisation of transduced cells in treated retina. Some adjacent cells were observed that were c-Fos positive cells but did not express OPN4 (or DsRed), which might indicate depolarisation of neighbouring cells and cell-to-cell signalling resulting from ectopic expression of OPN4 in the degenerate retina.

Data presented here demonstrate that a functional human melanopsin gene (OPN4) can be delivered to remaining retinal cells in a mouse model of end-stage retinal degeneration via subretinal injection of an AAV vector using a ubiquitous CBA promoter. Ectopically expressed OPN4 resulted in long-term restoration of the pupil light reflex and behavioural light avoidance up to the last time point tested at 13 months. Finally subretinal OPN4 expression led to light-induced changes in visual cortex blood flow and provided long-term improvements in a visually guided behavioural task indicating restoration of image-forming vision. In combination, these results suggest that this approach may be clinically useful in vision restoration in patients with end-stage RP.

In interpreting these data, a consideration is the mechanism by which visual responses were restored. The inventors believe that responses detected arise from activation of retinal circuitry involved in image-forming vision rather than augmenting existing ipRGCs for several reasons. Human melanopsin was not detected by immunohistochemistry in ganglion cell membranes in transduced retinae, and light-induced c-Fos expression in vector treated areas of retina was seen in multiple cells within the inner nuclear rather than the ganglion cell layer. MEA recordings revealed more responsive electrodes in treated retinas along with differences in firing rates and response kinetics compared to controls, suggesting that a wider range of ganglion cells were generating light-induced action potentials. The presence of visual cortex responses in treated mice and the restoration of ability to encode visual context also support activation of cells outside ipRGC pathways.

All publications mentioned in the above specification are herein incorporated by reference. Various modifications and variations of the described methods of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention, which are obvious to those skilled in biochemistry and biotechnology or related fields, are intended to be within the scope of the following claims.

## Claims

1. An adeno-associated virus (AAV) vector for use in treating an eye disease, or improving or restoring vision, wherein the AAV vector is administered to a mammalian subject by subretinal injection, and wherein the AAV vector comprises a nucleotide sequence encoding melanopsin.

2. The AAV vector for use according to claim 1, wherein object recognition is improved in the subject.

3. The AAV vector for use according to any preceding claim, wherein the eye disease is a retinal dystrophy or the subject suffers from a retinal dystrophy.

4. The AAV vector for use according to any preceding claim, wherein the eye disease is retinitis pigmentosa or the subject suffers from retinitis pigmentosa.

5. The AAV vector for use according to any preceding claim, wherein the eye to be treated has less than 50% of the functional rod and/or cone photoreceptor cells that were present prior to the onset of the disease.

6. The AAV vector for use according to any preceding claim, wherein the eye to be treated lacks rod and/or cone photoreceptor cells.

7. The AAV vector for use according to any preceding claim, wherein the AAV vector is in the form of an AAV vector particle.

8. The AAV vector for use according to any preceding claim, wherein the AAV vector is in the form of an AAV particle comprising an AAV8 Y733F mutant capsid.

9. The AAV vector for use according to any preceding claim, wherein the AAV vector comprises an AAV2 genome.

10. The AAV vector for use according to claim 7, wherein the AAV vector particle comprises an AAV2 genome and AAV8 capsid proteins (AAV2/8), preferably AAV8 Y733F mutant capsid proteins, or an AAV2 genome and AAV2 capsid proteins (AAV2/2).

11. The AAV vector for use according to any preceding claim, wherein the melanopsin is expressed in bipolar and/or horizontal cells.

12. The AAV vector for use according to any preceding claim, wherein the melanopsin is human melanopsin.

13. The AAV vector for use according to any preceding claim, wherein the melanopsin-encoding nucleotide sequence is selected from the group consisting of:
(a) a nucleotide sequence having at least 70% identity to SEQ ID NO: 1 or 2; and
(b) a nucleotide sequence encoding an amino acid sequence having at least 70% identity to SEQ ID NO: 3 or 4,
wherein the protein encoded by the nucleotide sequence substantially retains the natural function of the protein represented by SEQ ID NO: 3 or 4.

14. The AAV vector for use according to any preceding claim, wherein the nucleotide sequence encoding melanopsin is operably linked to a CBA promoter.

15. The AAV vector for use according to any preceding claim, wherein the subretinal injection comprises the steps:
(a) administering a solution to the subject by subretinal injection in an amount effective to at least partially detach the retina to form a subretinal bleb, wherein the solution does not comprise the AAV vector; and
(b) administering a medicament composition by subretinal injection into the bleb formed by step (a), wherein the medicament comprises the AAV vector.


SEQUENCE LISTING

<110>  Oxford University Innovation Limited

<120>  Treatment of eye disease

<130>  P110853EP

<160>  8

<170>  PatentIn version 3.5

<210>  1
<211>  1437
<212>  DNA
<213>  Artificial Sequence

<220>
<223>  nucleotide sequence encoding melanopsin

<400>  1
atgaaccctc cttcggggcc aagagtcccg cccagcccaa cccaagagcc cagctgcatg      60

gccaccccag caccacccag ctggtgggac agctcccaga gcagcatctc cagcctgggc     120

cggcttccat ccatcagtcc cacagcacct gggacttggg ctgctgcctg ggtccccctc     180

cccacggttg atgttccaga ccatgcccac tataccctgg gcacagtgat cttgctggtg     240

ggactcacgg ggatgctggg caacctgacg gtcatctata ccttctgcag gagcagaagc     300

ctccggacac ctgccaacat gttcattatc aacctcgcgg tcagcgactt cctcatgtcc     360

ttcacccagg cccctgtctt cttcaccagt agcctctata agcagtggct ctttggggag     420

acaggctgcg agttctatgc cttctgtgga gctctctttg gcatttcctc catgatcacc     480

ctgacggcca tcgccctgga ccgctacctg gtaatcacac gcccgctggc cacctttggt     540

gtggcgtcca agaggcgtgc ggcatttgtc ctgctgggcg tttggctcta tgccctggcc     600

tggagtctgc cacccttctt cggctggagc gcctacgtgc ccgaggggtt gctgacatcc     660

tgctcctggg actacatgag cttcacgccg gccgtgcgtg cctacaccat gcttctctgc     720

tgcttcgtgt tcttcctccc tctgcttatc atcatctact gctacatctt catcttcagg     780

gccatccggg agacaggacg ggctctccag accttcgggg cctgcaaggg caatggcgag     840

tccctgtggc agcggcagcg gctgcagagc gagtgcaaga tggccaagat catgctgctg     900

gtcatcctcc tcttcgtgct ctcctgggct ccctattccg ctgtggccct ggtggccttt     960

gctgggtacg cacacgtcct gacaccctac atgagctcgg tgccagccgt catcgccaag    1020

gcctctgcaa tccacaaccc catcatttac gccatcaccc accccaagta cagggtggcc    1080

attgcccagc acctgccctg cctgggggtg ctgctgggtg tatcacgccg gcacagtcgc    1140

ccctacccca gctaccgctc cacccaccgc tccacgctga ccagccacac ctccaacctc    1200

agctggatct ccatacggag gcgccaggag tccctgggct cggagagtga ggtgggctgg    1260

acacacatgg aggcagcagc tgtgtgggga gctgcccagc aagcaaatgg gcggtccctc 1320

tacggtcagg gtctggagga cttggaagcc aaggcacccc ccagacccca gggacacgaa 1380

gcagagactc cagggaagac caaggggctg atccccagcc aggaccccag gatgtag 1437

<210> 2
<211> 1470
<212> DNA
<213> Artificial Sequence

<220>
<223> nucleotide sequence encoding melanopsin

<400> 2
atgaaccctc cttcggggcc aagagtcccg cccagcccaa cccaagagcc cagctgcatg 60

gccaccccag caccacccag ctggtgggac agctcccaga gcagcatctc cagcctgggc 120

cggcttccat ccatcagtcc cacagcacct gggacttggg ctgctgcctg ggtccccctc 180

cccacggttg atgttccaga ccatgcccac tataccctgg gcacagtgat cttgctggtg 240

ggactcacgg ggatgctggg caacctgacg gtcatctata ccttctgcag agctgtgctt 300

cgtggagtca ctgtgatgat gcagagcaga agcctccgga cacctgccaa catgttcatt 360

atcaacctcg cggtcagcga cttcctcatg tccttcaccc aggcccctgt cttcttcacc 420

agtagcctct ataagcagtg gctctttggg gagacaggct gcgagttcta tgccttctgt 480

ggagctctct ttggcatttc ctccatgatc accctgacgg ccatcgccct ggaccgctac 540

ctggtaatca cacgcccgct ggccaccttt ggtgtggcgt ccaagaggcg tgcggcattt 600

gtcctgctgg gcgtttggct ctatgccctg gcctggagtc tgccaccctt cttcggctgg 660

agcgcctacg tgcccgaggg gttgctgaca tcctgctcct gggactacat gagcttcacg 720

ccggccgtgc gtgcctacac catgcttctc tgctgcttcg tgttcttcct ccctctgctt 780

atcatcatct actgctacat cttcatcttc agggccatcc gggagacagg acgggctctc 840

cagaccttcg gggcctgcaa gggcaatggc gagtccctgt ggcagcggca gcggctgcag 900

agcgagtgca agatggccaa gatcatgctg ctggtcatcc tcctcttcgt gctctcctgg 960

gctccctatt ccgctgtggc cctggtggcc tttgctgggt acgcacacgt cctgacaccc 1020

tacatgagct cggtgccagc cgtcatcgcc aaggcctctg caatccacaa ccccatcatt 1080

tacgccatca cccaccccaa gtacagggtg gccattgccc agcacctgcc ctgcctgggg 1140

gtgctgctgg gtgtatcacg ccggcacagt cgcccctacc ccagctaccg ctccacccac 1200

cgctccacgc tgaccagcca cacctccaac ctcagctgga tctccatacg gaggcgccag 1260

gagtccctgg gctcggagag tgaggtgggc tggacacaca tggaggcagc agctgtgtgg 1320

ggagctgccc agcaagcaaa tgggcggtcc ctctacggtc agggtctgga ggacttggaa 1380

gccaaggcac cccccagacc ccagggacac gaagcagaga ctccagggaa gaccaagggg 1440

ctgatcccca gccaggaccc caggatgtag                                          1470

<210>  3
<211>  478
<212>  PRT
<213>  Artificial Sequence

<220>
<223>  amino acid sequence of melanopsin

<400>  3

Met Asn Pro Pro Ser Gly Pro Arg Val Pro Pro Ser Pro Thr Gln Glu
1               5                   10                  15

Pro Ser Cys Met Ala Thr Pro Ala Pro Pro Ser Trp Trp Asp Ser Ser
            20                  25                  30

Gln Ser Ser Ile Ser Ser Leu Gly Arg Leu Pro Ser Ile Ser Pro Thr
        35                  40                  45

Ala Pro Gly Thr Trp Ala Ala Ala Trp Val Pro Leu Pro Thr Val Asp
    50                  55                  60

Val Pro Asp His Ala His Tyr Thr Leu Gly Thr Val Ile Leu Leu Val
65                  70                  75                  80

Gly Leu Thr Gly Met Leu Gly Asn Leu Thr Val Ile Tyr Thr Phe Cys
                85                  90                  95

Arg Ser Arg Ser Leu Arg Thr Pro Ala Asn Met Phe Ile Ile Asn Leu
            100                 105                 110

Ala Val Ser Asp Phe Leu Met Ser Phe Thr Gln Ala Pro Val Phe Phe
        115                 120                 125

Thr Ser Ser Leu Tyr Lys Gln Trp Leu Phe Gly Glu Thr Gly Cys Glu
    130                 135                 140

Phe Tyr Ala Phe Cys Gly Ala Leu Phe Gly Ile Ser Ser Met Ile Thr
145                 150                 155                 160

Leu Thr Ala Ile Ala Leu Asp Arg Tyr Leu Val Ile Thr Arg Pro Leu
                165                 170                 175

Ala Thr Phe Gly Val Ala Ser Lys Arg Arg Ala Ala Phe Val Leu Leu
            180                 185                 190

Gly Val Trp Leu Tyr Ala Leu Ala Trp Ser Leu Pro Pro Phe Phe Gly

195                 200                 205

Trp Ser Ala Tyr Val Pro Glu Gly Leu Leu Thr Ser Cys Ser Trp Asp
    210                 215                 220

Tyr Met Ser Phe Thr Pro Ala Val Arg Ala Tyr Thr Met Leu Leu Cys
225                 230                 235                 240

Cys Phe Val Phe Phe Leu Pro Leu Leu Ile Ile Ile Tyr Cys Tyr Ile
                245                 250                 255

Phe Ile Phe Arg Ala Ile Arg Glu Thr Gly Arg Ala Leu Gln Thr Phe
            260                 265                 270

Gly Ala Cys Lys Gly Asn Gly Glu Ser Leu Trp Gln Arg Gln Arg Leu
        275                 280                 285

Gln Ser Glu Cys Lys Met Ala Lys Ile Met Leu Leu Val Ile Leu Leu
    290                 295                 300

Phe Val Leu Ser Trp Ala Pro Tyr Ser Ala Val Ala Leu Val Ala Phe
305                 310                 315                 320

Ala Gly Tyr Ala His Val Leu Thr Pro Tyr Met Ser Ser Val Pro Ala
                325                 330                 335

Val Ile Ala Lys Ala Ser Ala Ile His Asn Pro Ile Ile Tyr Ala Ile
            340                 345                 350

Thr His Pro Lys Tyr Arg Val Ala Ile Ala Gln His Leu Pro Cys Leu
        355                 360                 365

Gly Val Leu Leu Gly Val Ser Arg Arg His Ser Arg Pro Tyr Pro Ser
    370                 375                 380

Tyr Arg Ser Thr His Arg Ser Thr Leu Thr Ser His Thr Ser Asn Leu
385                 390                 395                 400

Ser Trp Ile Ser Ile Arg Arg Arg Gln Glu Ser Leu Gly Ser Glu Ser
                405                 410                 415

Glu Val Gly Trp Thr His Met Glu Ala Ala Ala Val Trp Gly Ala Ala
            420                 425                 430

Gln Gln Ala Asn Gly Arg Ser Leu Tyr Gly Gln Gly Leu Glu Asp Leu
        435                 440                 445

Glu Ala Lys Ala Pro Pro Arg Pro Gln Gly His Glu Ala Glu Thr Pro
    450                 455                 460

Gly Lys Thr Lys Gly Leu Ile Pro Ser Gln Asp Pro Arg Met
465                 470                 475

<210>  4
<211>  489
<212>  PRT
<213>  Artificial Sequence

<220>
<223>  amino acid sequence of melanopsin

<400>  4

Met Asn Pro Pro Ser Gly Pro Arg Val Pro Pro Ser Pro Thr Gln Glu
1               5                   10                  15

Pro Ser Cys Met Ala Thr Pro Ala Pro Pro Ser Trp Trp Asp Ser Ser
            20                  25                  30

Gln Ser Ser Ile Ser Ser Leu Gly Arg Leu Pro Ser Ile Ser Pro Thr
        35                  40                  45

Ala Pro Gly Thr Trp Ala Ala Ala Trp Val Pro Leu Pro Thr Val Asp
    50                  55                  60

Val Pro Asp His Ala His Tyr Thr Leu Gly Thr Val Ile Leu Leu Val
65                  70                  75                  80

Gly Leu Thr Gly Met Leu Gly Asn Leu Thr Val Ile Tyr Thr Phe Cys
                85                  90                  95

Arg Ala Val Leu Arg Gly Val Thr Val Met Met Gln Ser Arg Ser Leu
            100                 105                 110

Arg Thr Pro Ala Asn Met Phe Ile Ile Asn Leu Ala Val Ser Asp Phe
        115                 120                 125

Leu Met Ser Phe Thr Gln Ala Pro Val Phe Phe Thr Ser Ser Leu Tyr
    130                 135                 140

Lys Gln Trp Leu Phe Gly Glu Thr Gly Cys Glu Phe Tyr Ala Phe Cys
145                 150                 155                 160

Gly Ala Leu Phe Gly Ile Ser Ser Met Ile Thr Leu Thr Ala Ile Ala
                165                 170                 175

Leu Asp Arg Tyr Leu Val Ile Thr Arg Pro Leu Ala Thr Phe Gly Val

            180                 185                 190

Ala Ser Lys Arg Arg Ala Ala Phe Val Leu Leu Gly Val Trp Leu Tyr
        195                 200                 205

Ala Leu Ala Trp Ser Leu Pro Pro Phe Phe Gly Trp Ser Ala Tyr Val
    210                 215                 220

Pro Glu Gly Leu Leu Thr Ser Cys Ser Trp Asp Tyr Met Ser Phe Thr
225                 230                 235                 240

Pro Ala Val Arg Ala Tyr Thr Met Leu Leu Cys Cys Phe Val Phe Phe
                245                 250                 255

Leu Pro Leu Leu Ile Ile Ile Tyr Cys Tyr Ile Phe Ile Phe Arg Ala
            260                 265                 270

Ile Arg Glu Thr Gly Arg Ala Leu Gln Thr Phe Gly Ala Cys Lys Gly
        275                 280                 285

Asn Gly Glu Ser Leu Trp Gln Arg Gln Arg Leu Gln Ser Glu Cys Lys
    290                 295                 300

Met Ala Lys Ile Met Leu Leu Val Ile Leu Leu Phe Val Leu Ser Trp
305                 310                 315                 320

Ala Pro Tyr Ser Ala Val Ala Leu Val Ala Phe Ala Gly Tyr Ala His
                325                 330                 335

Val Leu Thr Pro Tyr Met Ser Ser Val Pro Ala Val Ile Ala Lys Ala
            340                 345                 350

Ser Ala Ile His Asn Pro Ile Ile Tyr Ala Ile Thr His Pro Lys Tyr
        355                 360                 365

Arg Val Ala Ile Ala Gln His Leu Pro Cys Leu Gly Val Leu Leu Gly
    370                 375                 380

Val Ser Arg Arg His Ser Arg Pro Tyr Pro Ser Tyr Arg Ser Thr His
385                 390                 395                 400

Arg Ser Thr Leu Thr Ser His Thr Ser Asn Leu Ser Trp Ile Ser Ile
                405                 410                 415

Arg Arg Arg Gln Glu Ser Leu Gly Ser Glu Ser Glu Val Gly Trp Thr
            420                 425                 430

His Met Glu Ala Ala Ala Val Trp Gly Ala Ala Gln Gln Ala Asn Gly
        435                 440                 445

Arg Ser Leu Tyr Gly Gln Gly Leu Glu Asp Leu Glu Ala Lys Ala Pro
    450                 455                 460

Pro Arg Pro Gln Gly His Glu Ala Glu Thr Pro Gly Lys Thr Lys Gly
465                 470                 475                 480

Leu Ile Pro Ser Gln Asp Pro Arg Met
                485

<210>  5
<211>  738
<212>  PRT
<213>  adeno-associated virus 8

<400>  5

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Gln Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Arg Lys Arg Leu Asn Phe Gly Gln
                165                 170                 175

Thr Gly Asp Ser Glu Ser Val Pro Asp Pro Gln Pro Leu Gly Glu Pro
            180                 185                 190

Pro Ala Ala Pro Ser Gly Val Gly Pro Asn Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ala Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Ser Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Gln Phe Thr Tyr
                405                 410                 415

Thr Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Thr Thr Gly Gly Thr Ala Asn Thr Gln Thr Leu Gly
    450                 455                 460

Phe Ser Gln Gly Gly Pro Asn Thr Met Ala Asn Gln Ala Lys Asn Trp
465                 470                 475                 480

Leu Pro Gly Pro Cys Tyr Arg Gln Gln Arg Val Ser Thr Thr Thr Gly
                485                 490                 495

Gln Asn Asn Asn Ser Asn Phe Ala Trp Thr Ala Gly Thr Lys Tyr His
            500                 505                 510

Leu Asn Gly Arg Asn Ser Leu Ala Asn Pro Gly Ile Ala Met Ala Thr
        515                 520                 525

His Lys Asp Asp Glu Glu Arg Phe Phe Pro Ser Asn Gly Ile Leu Ile
    530                 535                 540

Phe Gly Lys Gln Asn Ala Ala Arg Asp Asn Ala Asp Tyr Ser Asp Val
545                 550                 555                 560

Met Leu Thr Ser Glu Glu Glu Ile Lys Thr Thr Asn Pro Val Ala Thr
                565                 570                 575

Glu Glu Tyr Gly Ile Val Ala Asp Asn Leu Gln Gln Gln Asn Thr Ala
            580                 585                 590

Pro Gln Ile Gly Thr Val Asn Ser Gln Gly Ala Leu Pro Gly Met Val
        595                 600                 605

Trp Gln Asn Arg Asp Val Tyr Leu Gln Gly Pro Ile Trp Ala Lys Ile
    610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Asn Gln Ser Lys Leu Asn Ser Phe
            660                 665                 670

Ile Thr Gln Tyr Ser Thr Gly Gln Val Ser Val Glu Ile Glu Trp Glu
        675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Ser Val Asp Phe Ala Val Asn Thr Glu
705                 710                 715                 720

Gly Val Tyr Ser Glu Pro Arg Pro Ile Gly Thr Arg Tyr Leu Thr Arg
                725                 730                 735

Asn Leu

<210>  6
<211>  934
<212>  DNA
<213>  Artificial Sequence

<220>
<223>  promoter sequence

<400>  6
attgacgtca ataatgacgt atgttcccat agtaacgcca atagggactt tccattgacg      60

tcaatgggtg gagtatttac ggtaaactgc ccacttggca gtacatcaag tgtatcatat     120

gccaagtacg ccccctattg acgtcaatga cggtaaatgg cccgcctggc attatgccca     180

gtacatgacc ttatgggact ttcctacttg gcagtacatc tacgtattag tcatcgctat     240

taccatggtc gaggtgagcc ccacgttctg cttcactctc cccatctccc ccccctcccc     300

acccccaatt ttgtatttat ttatttttta attattttgt gcagcgatgg gggcgggggg     360

gggggggggg cgcgcgccag gcggggcggg gcggggcgag gggcggggcg gggcgaggcg     420

gagaggtgcg gcggcagcca atcagagcgg cgcgctccga aagtttcctt ttatggcgag     480

gcggcggcgg cggcggccct ataaaaagcg aagcgcgcgg cgggcgggag tcgctgcgcg     540

ctgccttcgc cccgtgcccc gctccgccgc cgcctcgcgc cgcccgcccc ggctctgact     600

gaccgcgtta ctcccacagg tgagcgggcg ggacggccct tctcctccgg gctgtaatta     660

gcgcttggtt taatgacggc ttgtttcttt tctgtggctg cgtgaaagcc ttgaggggct     720

ccgggagggc cctttgtgcg gggggagcgg ctcggggctg tccgcggggg gacggctgcc     780

ttcggggggg acggggcagg gcggggttcg gcttctggcg tgtgaccggc ggctctagag     840

cctctgctaa ccatgttcat gccttcttct ttttcctaca gctcctgggc aacgtgctgg     900

ttattgtgct gtctcatcat tttggcaaag aatt                                 934

<210>  7
<211>  270
<212>  DNA
<213>  Artificial Sequence

<220>
<223>  Bovine Growth Hormone poly-A signal

<400>  7
tcgctgatca gcctcgactg tgccttctag ttgccagcca tctgttgttt gcccctcccc      60

cgtgccttcc ttgaccctgg aaggtgccac tcccactgtc ctttcctaat aaaatgagga     120

aattgcatcg cattgtctga gtaggtgtca ttctattctg gggggtgggg tggggcagga     180

cagcaagggg gaggattggg aagacaatag caggcatgct ggggatgcgg tgggctctat     240

ggcttctgag gcggaaagaa ccagctgggg                                      270


<210>  8
<211>  588
<212>  DNA
<213>  Artificial Sequence

<220>
<223>  example sequence of the woodchuck hepatitis post-transcriptional
       regulatory element (WPRE)

<400>  8
atcaacctct ggattacaaa atttgtgaaa gattgactgg tattcttaac tatgttgctc      60

cttttacgct atgtggatac gctgctttaa tgcctttgta tcatgctatt gcttcccgta     120

tggctttcat tttctcctcc ttgtataaat cctggttgct gtctctttat gaggagttgt     180

ggcccgttgt caggcaacgt ggcgtggtgt gcactgtgtt tgctgacgca acccccactg     240

gttggggcat tgccaccacc tgtcagctcc tttccgggac tttcgctttc cccctcccta     300

ttgccacggc ggaactcatc gccgcctgcc ttgcccgctg ctggacaggg gctcggctgt     360

tgggcactga caattccgtg gtgttgtcgg ggaaatcatc gtcctttcct tggctgctcg     420

cctgtgttgc cacctggatt ctgcgcggga cgtccttctg ctacgtccct tcggccctca     480

atccagcgga ccttccttcc cgcggcctgc tgccggctct gcggcctctt ccgcgtcttc     540

gccttcgccc tcagacgagt cggatctccc tttgggccgc ctccccgc                  588